# FASCICULE DE BREVET EUROPEEN

(11) **EP 1 165 573 B1**
(45) Date de publication et mention de la délivrance du brevet: **21.01.2004**
(21) Numéro de dépôt: 00917119.0
(22) Date de dépôt: 04.04.2000
(51) Int. Cl.: C07F 9/40, A61K 31/66, C07F 9/655, C12N 5/06

(54) **COMPOSES INHIBITANT SELECTIVEMENT LES LYMPHOCYTES T GAMMA 9 DELTA 2**
VERBINDUNGEN SELEKTIF-INHIBITOREN DER T GAMMA 9 DELTA 2 LYMPHOCYTEN
COMPOUNDS SELECTIVELY INHIBITING GAMMA 9 DELTA 2 T LYMPHOCYTES

(30) Priorité: 06.04.1999 FR 9904263
(43) Date de publication de la demande: 02.01.2002
(73) Titulaire: INSTITUT NATIONAL DE LA SANTE ET DE LA RECHERCHE MEDICALE (INSERM), 75013 Paris (FR); SANGSTAT MEDICAL CORPORATION, Fremont, California 94555 (US)
(72) Inventeur: BELMANT, Christian, F-31700 Blagnac (FR); BONNEVILLE, Marc, F-44120 Vertou (FR); PEYRAT, Marc, Alix, F-44230 St. Sebastien (FR); FOURNIE, Jean-Jacques, F-31450 Corronsac (FR); KOZIKOWSKI, Alan, P., Princetown, NJ 08540 (US)
(74) Mandataire: Cabinet BARRE LAFORGUE & associés
(86) Numéro de dépôt international: PCT/FR2000/000837
(87) Numéro de publication internationale: WO 2000/059916

(56) Documents cités:
- WO-A-00/12516
- WO-A-00/12519
- WO-A-95/20673
- US-A- 5 639 653
- SCHOEL B ET AL: "Phosphate is essential for stimulation of V.gamma.9V.delta.2 T lymphocytes by mycobacterial low molecular weight ligand" EUROPEAN JOURNAL OF IMMUNOLOGY, vol. 24, no. 8, 1994, pages 1886-1892, XP002102421 ISSN: 0014-2980
- GOTOH T ET AL: "Different roles of the diphosphate moieties of allylic and homoallylic diphosphates in the prenyltransferase reaction" BIOCHEM. BIOPHYS. RES. COMMUN. (BBRCA9,0006291X);1988; VOL.156 (1); PP.396-402, XP002118543 Tohoku Univ.;Chem. Res. Inst. Non-Aqueous Solutions; Sendai; 980; Japan (JP)
- V. JO DAVISSON: "Phosphorylation of isoprenoid alcohols" JOURNAL OF ORGANIC CHEMISTRY., vol. 51, no. 25, 1986, pages 4768-4779, XP002118544 AMERICAN CHEMICAL SOCIETY. EASTON., US ISSN: 0022-3263 cité dans la demande

## Description

L'invention concerne des composés inhibant sélectivement les lymphocytes Tγ9δ2 porteurs de récepteurs à régions variables Vγ9 et Vδ2.

Les lymphocytes Tγδ des primates présents dans le sang périphérique (humains, singes) représentent, chez l'individu sain, habituellement de 1 à 5% des lymphocytes du sang et jouent un rôle dans le système immunitaire. Il a été démontré qu'ils reconnaissent leurs ligands antigéniques par une interaction directe avec l'antigène, sans présentation par les molécules du CMH d'une cellule présentatrice. Les lymphocytes Tγ9δ2 (parfois aussi désignés lymphocytes Tγ2δ2) sont des lymphocytes Tγδ porteurs de récepteurs TCR à régions variables Vγ9 et Vδ2. Ils représentent la majorité des lymphocytes Tγδ dans le sang humain.

Lorsqu'ils sont activés, les lymphocytes Tγδ exercent une puissante activité cytotoxique non restreinte par le CMH, particulièrement efficace pour tuer divers types de cellules, notamment des cellules pathogènes. Néanmoins, l'activation massive des lymphocytes Tγδ accompagnant parfois le développement de certaines pathologies peut présenter ou induire un caractère pathogène. Tel est le cas en particulier des maladies auto-immunes telles que la sclérose en plaques (Wucherpfennig K. *et al* "γδT cell receptor repertoire in acute multiple scerosis lesion" 1992, PNAS 89, 4588) ou la maladie de Behçet (Yamashita N. *et al* "Role of γδT lymphocytes in the development of Behçet disease" Clinical Experimental, Immunology, 107(2), 241-247).

Tel est la cas en outre d'un certain nombre de pathologies bactériennes telles que la brucellose, la tularémie, les salmonelloses, les tuberculoses, l'ehrlichiose, ou parasitaires telles que la malaria (accès de paludisme), la leishmaniose viscérale, la toxoplasmose (par exemple Morita C.T. *et al*, "Direct présentation of non peptide prenyl pyrophosphate antigens to human gamma delta T cells", 1996, Research in Immunology, Vol. 147, p 347-353).

Divers antigènes des lymphocytes Tγ9δ2 ont été décrits (WO-9520673, US-5639653, "Natural and synthetic non peptide antigens recognized by human γδT cells", Yoshimasa Tanaka *et al,* Nature, 375, 1995, pp 155-158). Néanmoins, ces antigènes naturels ne sont pas complètement identifiés. En outre, on sait que le mécanisme de l'activation des lymphocytes Tγ9δ2 par ces antigènes est particulier puisqu'il n'implique aucune molécule connue du CMH (complexe majeur d'histocompatibilité). Mais, la nature de ce mécanisme reste inexpliquée, de sorte que le problème de la mise au point d'inhibiteurs des lymphocytes Tγ9δ2 reste posé.

WO-9520673 indique aussi que les principes présentant une activité enzymatique phosphatase (monoster phosphorique phosphohydrolase et/ou nucléotide pyrophosphatase et/ou diester phosphorique phosphohydrolase) tels que la phosphatase alcaline, sont susceptibles d'inhiber l'activité d'antigènes d'origine naturelle, dits TUBag, issus d'un extrait mycobactérien, vis-à-vis des lymphocytes Tγ9δ2. Néanmoins, cette inhibition intervient par clivage des antigènes et n'agit donc pas sur les lymphocytes Tγ9δ2 eux-mêmes. En outre, elle est non spécifique et pose des problèmes d'effets secondaires incontrôlables dans la mesure où les milieux biologiques ou physiologiques comprennent eux-mêmes de nombreux composés phosphorylés et des activités enzymatiques phosphatases naturelles.

L'invention vise donc à proposer des composés d'inhibition sélective de la stimulation lymphocytaire Tγ9δ2, c'est-à-dire des composés immunosupresseurs spécifiques des lymphocytes Tγ9δ2.

L'invention vise plus particulièrement à proposer de tels composés qui soient compatibles, d'une part, avec une administration à un primate, et, d'autre part, avec les contraintes de rentabilité d'une exploitation industrielle (qui puissent être fabriqués de façon simple, en grandes quantités, à un coût acceptable à l'échelle industrielle).

Par ailleurs, il est aussi souhaitable que l'inhibition des lymphocytes Tγ9δ2 pour le traitement d'un excès d'activation de ces lymphocytes Tγ9δ2 ne détruise pas définitivement le système immunitaire du patient ou du milieu biologique lymphocytaire. Ainsi, l'invention vise aussi à proposer des composés ayant une activité d'inhibition qui soit non seulement sélective à l'égard des lymphocytes Tγ9δ2, mais également réversible, de sorte que l'activité des lymphocytes Tγ9δ2 puisse ultérieurement être restaurée.

L'invention vise également à proposer de nouveaux composés phosphorés à titre de substances thérapeutiquement actives.

L'invention vise aussi à proposer des applications des composés selon l'invention pour l'inhibition sélective et réversible des lymphocytes Tγ9δ2, dans un milieu extra-corporel. Plus particulièrement l'invention vise à proposer des composés pouvant être appliqués à titre thérapeutique, ou pour le diagnostic, et/ou pour l'étude expérimentale des lymphocytes Tγ9δ2, de leurs antigènes ou agents immunosuppresseurs spécifiques.

L'invention vise en particulier à proposer des composés susceptibles d'être utilisés dans un traitement des pathologies impliquant une activation des lymphocytes Tγ9δ2, et notamment choisie parmi la malaria (accès de paludisme), la leishmaniose viscérale, la toxoplasmose, la brucellose, la tularémie, les salmonelloses, les tuberculoses, l'ehrlichiose, les maladies auto-immunes telles que la sclérose en plaques ou la maladie de Behçet.

Pour ce faire, l'invention concerne de nouveaux composés conformes à la revendication 1.

L'invention concerne également des composés de formule:

CH₃―R₁―(CH₂)₂―R₂ (I)

où R₁ est choisi parmi les fonctions suivantes : 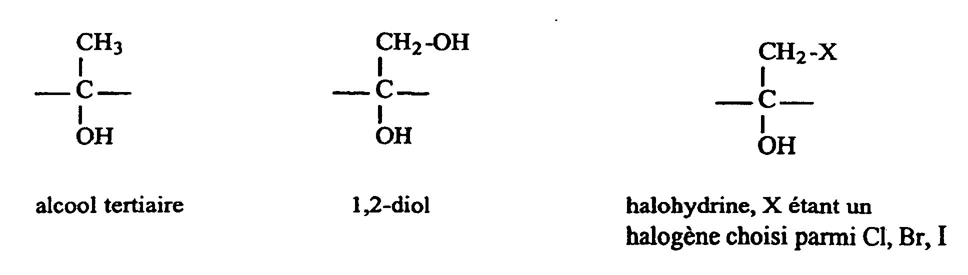 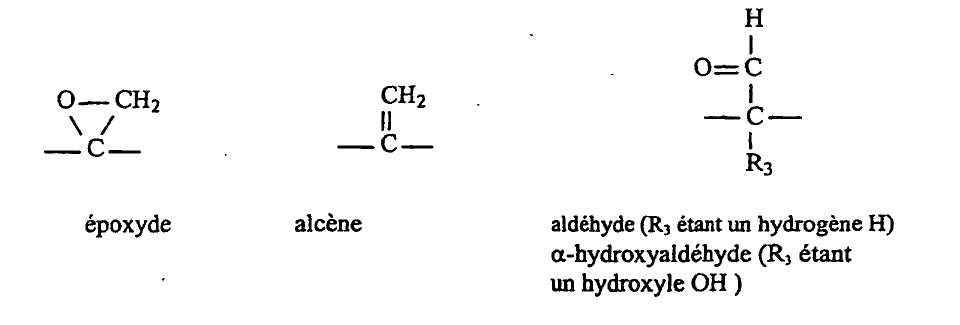 et R₂ est choisi parmi l'un des groupements suivants : 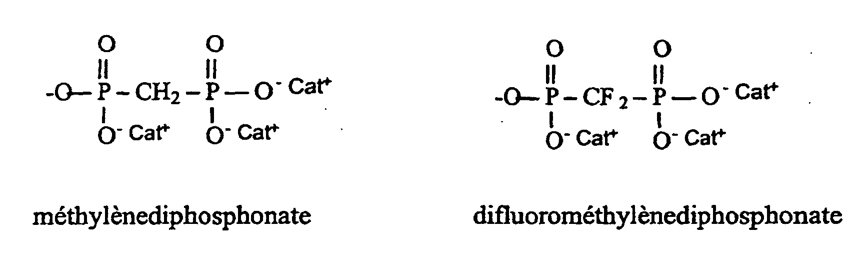 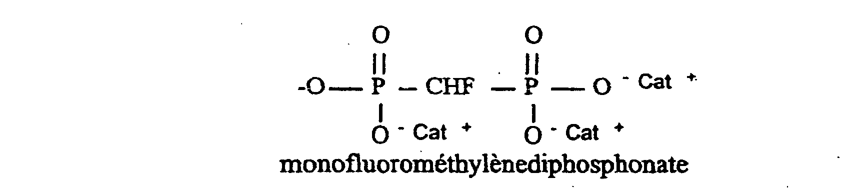 où Cat+ représente un (ou des) cation(s) organique(s) ou minéral(aux) (y compris le proton) identiques ou différents dans le même composé, pour leur utilisation à titre de substances thérapeutiquement actives.

Les composés conformes à la formule (I) de l'invention sont .les suivants (nomenclature IUPAC) :
R₁ : fonction alcool tertiaire:
   3-méthyl-3-butanol-1-yl-méthylènediphosphonate ;
   3-méthyl-3-butanol-1-yl-monofluorométhylènediphosphonate;
   3-méthyl-3-butanol-1-yl-difluorométhylènediphosphonate ;
R₁ : fonction 1,2-diol :
   3-méthyl-3,4-butanediol-1-yl-difluorométhylènediphosphonate.
R₁ : fonction halohydrine avec X = Cl, Br, I :
   3-(chlorométhyl)-3-butanol-1-yl-méthylènediphosphonate;
   3-(chlorométhyl)-3-butanol-1-yl-monofluorométhylènediphosphonate;
   3-(chlorométhyl)-3-butanol-1-yl-difluorométhylènediphosphonate;
   3-(bromométhyl)-3-butanol-1-yl-méthylène-diphosphonate ;
   3-(bromométhyl)-3-butanol-1-yl-monofluorométhylènediphosphonate;
   3-(bromométhyl)-3-butanol-1-yl-difluorométhylène-diphosphonate ;
   3-(iodométhyl)-3-butanol-1-yl-monofluorométhylènediphosphonate ;
   3-(iodométhyl)-3-butanol-1-yl-méthylènediphosphonate ;
   3-(iodométhyl)-3-butanol-1-yl-difluoro-méthylènediphosphonate.
R₁ : fonction époxyde :
   3,4-époxy-3-méthyl-1-butyl-méthylènediphosphonate ;
   3,4-époxy-3-méthyl-1-butyl-monofluorométhylènediphosphonate ;
   3,4-époxy-3-méthyl-1-butyl-difluorométhylènediphosphonate.
R₁ : fonction alcène :
   3-méthyl-3-butène-1-yl-méthylènediphosphonate ;
   3-méthyl-3-butène-1-yl-monofluorométhylènediphosphonate ;
   3-methyl-3-butène-1-yl-difluorométhylènediphosphonate.
R₁ : fonction aldéhyde (R₃ = H):
   3-formyl-1-butyl-méthylènediphosphonate ;
   3-formyl-1-butyl-monofluorométhylènediphosphonate ;
   3-formyl-1-butyl-difluorométhylènediphosphonate.
R₁ : fonction α-hydroxyaldéhyde (R₃ = OH) :
   3-formyl-3-butanol-1-yl-méthylènediphosphonate ;
   3-formyl-3-butanol-1-yl-monofluorométhylènediphosphonate ;
   3-formyl-3-butanol-1-yl-difluorométhylènediphosphonate.

Le 3-méthyl-3-butène-1-yl-diflurométhylènediphosphonate a été décrit par "phosphorylation of isoprenoid alcohols" V. Jo Davisson *et al.,* J. Org. Chem. 1986, 51, 4775.

L'invention concerne en outre les composés de formule (I) ci-dessus pour leurs utilisations à titre de substances thérapeutiquement actives - notamment comme agents d'inhibition sélective des lymphocytes Tγ9δ2-.

L'invention concerne plus particulièrement les composés de formule (I) ci-dessus pour leurs utilisations comme agents d'inhibition de l'activation sélective phosphoantigénique des lymphocytes Ty9δ2 par un agent antigène phosphaté (phosphoantigène), tel qu'un antigène naturel (par exemple les Tubag décrits par WO-9520673), ou artificiel tel que l'IPP (3-méthyl-3-butène-1-yl-pyrophosphate), un composé phosphohalohydrine tel que BrHPP (3-(bromométhyl)-3-butanol-1-yl-diphosphate) ou IHPP (3-(iodométhyl)-3-butanol-1-yl-diphosphate), ou un composé phosphoépoxyde tel que EpoxPP (3,4 époxy-3-méthyl-1-butyl-diphosphate).

Bien que le mécanisme réel de l'inhibition des lymphocytes Tγ9δ2 par les composés de l'invention ne soit pas définitivement élucidé, les travaux des inventeurs permettent de penser qu'une telle inhibition sélective des lymphocytes Tγ9δ2 peut être obtenue par des composés qui satisfont les trois conditions suivantes :
1) présenter une molécule de forme topologique correspondant à la formule (I),
2) présenter une fonction R₁ susceptible de former une liaison covalente suite à une réaction du type substitution ou addition nucléophile, ou addition électrophile en présence des lymphocytes Tγ9δ2,
3) présenter un groupement analogue structural d'un pyrophosphate, mais susceptible d'inhiber l'hydrolyse enzymatique du phosphate terminal nécessaire à l'activation des lymphocytes Tγ9δ2.

Un tel composé peut en effet avoir la propriété d'occuper les sites de reconnaissance antigénique des récepteurs Vγ9 Vδ2 grâce aux conditions 1) et 2), mais d'empêcher la transduction du signal d'activation au lymphocyte du fait que l'hydrolyse enzymatique du phosphate terminal, dont les inventeurs pensent qu'elle serait nécessaire à cette transduction, est inhibée.

La fonction R₁ est choisie de façon à être compatible avec les conditions 1) et 2) ci-dessus et pour permettre l'obtention du composé selon l'invention. Le groupement CH₃―R₁―(CH₂)₂― doit ainsi être un ligand antigénique du récepteur T Vγ9 Vδ2. Il peut s'agir de l'isopentényl, bien connu, qui est un ligand antigénique. Les inventeurs ont démontré en outre que les autres groupements CH₃―R₁―(CH₂)₂― de la formule (I) mentionnés ci-dessus permettent aussi d'obtenir des inhibiteurs des lymphocytes Tγ9δ2.

Le groupement R₂ est choisi parmi les analogues structuraux des pyrophosphates non hydrolysables ou faiblement hydrolysables De tels analogues des pyrophosphates sont connus en eux-mêmes (cf. "ATP analogs" par R.G. Yount (1975) Adv. Enzymol. Vol. 43, p 1-56 ; « Synthesis of monofluoro- and difluoro- methylenephosphonate analogues of sn-glycerol-3-phosphate as substrates for glycerol-3-phosphate dehydrogenase and the X-Ray structure of the fluorométhylenephosphonate moiety » par J. Nieschalk et *al.* (1996) Tetrahedron vol. 52 p165-176 ; "The difluoromethylenephosphonate moiety as a phosphate mimic : X ray structure of 2 amino-1,1-difluoro ethylphosphonic acid" par R.D. Chambers *et al.* (1990) J. Chem. Soc. Chem. Commun. vol. 15, p 1053-1054).

Il convient en outre de choisir un groupement R₂ compatible avec la synthèse du composé selon l'invention.

L'invention s'étend également aux utilisations des composés selon l'invention à titre d'inhibiteurs des lymphocytes Tγ9δ2 des primates, notamment à titre d'inhibiteur de la prolifération et/ou de l'activité cytotoxique et/ou de la production de substance(s) médiatrice(s) par les lymphocytes Tγ9δ2 des primates à récepteurs TCR comprenant les régions variables Vγ9 et Vδ2.

Les composés selon l'invention peuvent être appliqués pour le traitement de cellules sensibles aux lymphocytes Tγ9δ2 des primates, dans un milieu naturel ou artificiel susceptible de contenir des lymphocytes Tγ9δ2, dans lequel lesdites cellules peuvent être mises en contact avec ces lymphocytes Tγ9δ2, ce milieu étant compatible avec les composés selon l'invention (c'est-à-dire n'est pas susceptible d'en provoquer la dégradation au moins dans certaines conditions du traitement).

Par "cellule sensible aux lymphocytes Tγ9δ2", on entend toute cellule sujette à l'activité effectrice induite des lymphocytes Tγ9δ2 (mort cellulaire, l'invention permettant d'empêcher la destruction de ces cellules par les lymphocytes); réception de médiateurs relargués par les lymphocytes Tγ9δ2 (TNF-α, INF-γ...) ; prolifération cellulaire induite par les lymphocytes Tγ9δ2.

L'invention s'étend donc à un procédé d'inhibition sélective des lymphocytes Tγ9δ2 -notamment à un procédé d'inhibition sélective de la prolifération des lymphocytes Tγ9δ2 et/ou de l'activité cytotoxique des lymphocytes Tγ9δ2 et/ou de la production de substance(s) médiatrice(s) par les lymphocytes Tγ9δ2- dans lequel on met ces lymphocytes Tγ9δ2 au contact d'au moins un composé selon l'invention dans un milieu contenant des lymphocytes Tγ9δ2.

Avantageusement et selon l'invention, on utilise au moins un composé selon l'invention à une concentration dans le milieu qui procure une inhibition sélective de la prolifération polyclonale des lymphocytes Tγ9δ2. Ce milieu peut être choisi parmi le sang humain, le sang d'un primate non humain, les extraits de sang humain, et les extraits de sang d'un primate non humain.

Avantageusement et selon l'invention, on utilise une concentration supérieure à la concentration IC50 du composé selon l'invention, définie comme celle permettant de réduire de 50% l'intensité de la réponse des lymphocytes Tγ9δ2, selon le test de cytotoxicité induite, à un stimulant antigénique étalon, notamment le BrHPP à 80nM.

Ledit milieu peut être extracorporel, ledit procédé d'inhibition selon l'invention étant alors un traitement extracorporel, pouvant notamment servir en laboratoire, par exemple pour le diagnostic ou l'étude des lymphocytes Tγ9δ2 ou de leurs propriétés. Pour le diagnostic, l'inhibition des lymphocytes Tγ9δ2 peut servir à évaluer l'état d'activation des lymphocytes Tγ9δ2 prélevés sur un patient, selon leur comportement après mise au contact d'une quantité inhibitrice d'un composé selon l'invention.

Ledit milieu peut être aussi intracorporel, l'inhibition sélective des lymphocytes Tγ9δ2 ayant alors une utilité thérapeutique ou de diagnostic.

Plus particulièrement, ledit milieu est le sang périphérique d'un primate. L'invention s'étend donc en particulier à un procédé d'inhibition sélective des lymphocytes Tγ9δ2 du sang périphérique d'un primate -notamment de l'homme- dans lequel on administre une quantité apte à inhiber les lymphocytes Tγ9δ2 d'au moins un composé selon l'invention. On administre donc au moins un composé selon l'invention par voie générale -notamment parentérale dans le sang périphérique-.

Ledit milieu peut aussi être un site cellulaire à traiter, et on administre au moins un composé selon l'invention directement au contact du site cellulaire à traiter (administration topique).

Ainsi, les composés selon l'invention peuvent être appliqués à titre thérapeutique pour le traitement curatif ou préventif des pathologies induisant une activation des lymphocytes Tγ9δ2 des primates dans un milieu pouvant contenir ces lymphocytes Tγ9δ2.

L'invention concerne donc aussi les composés de la formule (I) pour leur utilisation à titre de substances thérapeutiquement actives chez les primates. L'invention concerne aussi les composés selon la formule (I), pour leur utilisation dans une composition thérapeutique destinée à être administrée à un primate en vue du traitement préventif ou curatif d'une pathologie impliquant l'activation des lymphocytes Tγ9δ2.

Les composés selon l'invention peuvent être avantageusement utilisés dans le cadre du traitement des pathologies des primates appartenant au groupe formé des parasitoses choisies parmi la malaria (paludisme), la leishmaniose viscérale et la toxoplasmose ; des maladies auto-immunes -notamment la sclérose en plaques et la maladie de Behçet- induisant une activation des lymphocytes Tγ9δ2 ; des pathologies bactériennes choisies parmi la brucellose, la tularémie, les salmonelloses, les tuberculoses, et l'ehrlichiose. On administre alors une composition thérapeutique adaptée pour libérer dans le sang périphérique et/ou sur un site cellulaire à traiter une quantité d'au moins un composé selon l'invention apte à inhiber les lymphocytes Tγ9δ2.

En effet, il a été démontré de façon générale dans l'art antérieur sus-cité qu'une composition ayant la propriété d'inhiber les lymphocytes Tγ9δ2 peut être avantageusement utilisée pour le traitement de ces pathologies.

De façon traditionnelle, dans tout le texte, les termes "thérapie" ou "thérapeutique" englobent non seulement les traitements curatifs ou les soins, mais également les traitements préventifs (prophylaxie) tels que la vaccination. En effet, en permettant l'inhibition sélective des lymphocytes Tγ9δ2, l'invention permet des traitements d'immunostimulation pouvant être avantageux aussi bien à titre prophylactique en empêchant le développement des lymphocytes Tγ9δ2, qu'à titre curatif en inhibant les lymphocytes Tγ9δ2.

L'invention s'étend donc aussi à une composition thérapeutique ou de diagnostic comprenant au moins un composé selon l'invention. Plus particulièrement, l'invention concerne une composition thérapeutique comprenant une quantité apte à être administrée à un primate -notamment au contact du sang périphérique ou par voie topique d'au moins un composé selon l'invention -notamment pour le traitement préventif ou curatif des pathologies suscitées-. Une composition selon l'invention peut être une composition immunostimulante, ou un vaccin, les composés selon l'invention étant des antigènes inhibant sélectivement les lymphocytes Tγ9δ2.

Une composition thérapeutique selon l'invention peut être préparée sous une forme galénique apte à être administrée par voie générale, notamment par voie parentérale directement dans le sang périphérique d'un primate, avec au moins un composé selon l'invention en quantité adaptée pour inhiber les lymphocytes Tγ9δ2 et un ou plusieurs excipient(s) approprié(s). Compte tenu de la concentration active des composés selon l'invention (de l'ordre de 10 à 1000 µM), une telle administration est envisageable sans risque de toxicité.

Une composition thérapeutique selon l'invention peut aussi être préparée sous une forme galénique appropriée pour son administration topique, directement au contact des lymphocytes Tγ9δ2.

La forme galénique d'une composition thérapeutique selon l'invention est réalisée selon la voie d'administration choisie, par les techniques traditionnelles de formulation galénique. La quantité et la concentration de composé(s) selon l'invention, et la posologie sont déterminées par référence aux traitements chimiothérapeutiques connus des maladies à traiter, compte tenu de la bioactivité des composés selon l'invention vis-à-vis des lymphocytes Tγ9δ2, de l'individu à traiter, de la maladie concernée et des effets biologiques recherchés.

Une composition thérapeutique selon l'invention contient avantageusement une quantité d'au moins un composé selon l'invention adaptée pour créer dans le sang périphérique du patient une concentration supérieure à la concentration IC50 du(des) composé(s) selon l'invention telle que définie ci-dessus.

Avantageusement, pour un composé bioactif à une concentration comprise entre 1µM et 1000µM, une administration par voie générale d'une quantité de composé(s) selon l'invention comprise entre 0,1mg et 1g - notamment entre 1mg et 100mg- par kilogramme de poids du patient, est conseillée.

Par ailleurs, il a été démontré in vitro que les composés selon l'invention ne présentent aucune toxicité générale. En outre, on sait que la catégorie biochimique de molécules à laquelle les composés selon l'invention appartiennent (phosphoesters) constitue une famille de composés compatibles avec les milieux biologiques analogues et physiologiques. Les composés selon l'invention ne présentent donc pas d'autres effets toxiques que ceux induits par leur bioactivité sur les lymphocytes Tγ9δ2.

En outre, les composés selon l'invention présentent un poids moléculaire suffisamment faible (notamment inférieur à 500) pour être compatible avec leur élimination par voie rénale et urinaire.

Un exemple de formulation de composition thérapeutique injectable selon l'invention pour un primate de 1kg est le suivant :
5 mg de sels de sodium du 3,4-époxy-3-méthyl-1-butylméthylènediphosphonate (Epox-PCP) dilués dans 5 ml de tampon Ringer -Lactate stérile.

On administre ainsi pendant 4 jours: 1 dose par jour de 5 mg pour 1kg d'animal, correspondant à une concentration dans le sang circulant de 50 mg/l, adaptée pour être supérieure à la concentration IC50 de 15 µM pour l' Epox-PCP (une concentration de 50mg/l correspondant à environ 160 µM).

Il est à noter que la majorité des excipients ou autres additifs pharmaceutiquement acceptables traditionnellement utilisés, sont chimiquement compatibles avec les composés selon l'invention.

Une composition thérapeutique selon l'invention peut aussi avantageusement comprendre un ou plusieurs autre(s) principe(s) actif(s), notamment pour procurer un effet synergique. En particulier, un composé selon l'invention peut faire office d'adjuvant de vaccin. La composition thérapeutique vaccinante selon l'invention est alors formée d'une composition vaccinante connue à laquelle on rajoute une quantité de composé(s) selon l'invention apte à inhiber les lymphocytes Tγ9δ2 qui ne pourront exercer ni leur activité effectrice directe (par exemple cytotoxique), ni régulatrice de type Th-1 (par exemple relargage d'interféron et de facteur de nécrose tumorale (TNF ou "tumor necrosis factor")), et favorisent ainsi les réponses des lymphocytes B (par exemple production d'anticorps).

L'invention s'étend aussi à l'utilisation d'au moins un composé selon l'invention pour la fabrication d'une composition thérapeutique selon l'invention. Plus particulièrement, l'invention porte sur l'utilisation d'au moins un composé selon l'invention pour la fabrication d'une composition thérapeutique destinée au traitement préventif ou curatif d'une pathologie induisant une activation des lymphocytes Tγ9δ2 des primates -notamment une pathologie sélectionnée dans le groupe mentionné ci-dessus-. A ce titre, l'invention s'étend aussi à l'utilisation d'au moins un composé selon l'invention pour la fabrication d'une composition thérapeutique destinée à être administrée -notamment au contact du sang périphérique ou par voie topique- à un primate -notamment à l'homme- pour le traitement préventif ou curatif d'une pathologie telle que mentionnée ci-dessus.

L'invention s'étend aussi à un procédé de fabrication d'une composition -notamment une composition thérapeutique- selon l'invention ayant la propriété d'inhiber sélectivement les lymphocytes Tγ9δ2, dans lequel on utilise au moins un composé selon l'invention. L'invention porte aussi sur un procédé de fabrication d'une composition thérapeutique destinée au traitement préventif ou curatif d'une pathologie telle que mentionné ci-dessus, dans lequel on utilise au moins un composé selon l'invention. L'invention porte en particulier sur un procédé de fabrication d'une composition thérapeutique destinée à être administrée - notamment au contact du sang périphérique ou par voie topique- à un primate pour le traitement préventif ou curatif d'une pathologie telle que mentionnée ci-dessus-, dans lequel on utilise au moins un composé selon l'invention.

Les composés selon l'invention peuvent être préparés selon les schémas réactionnels donnés ci-après, selon les différents groupements R1 et R2.

Dans ces schémas, PCP identifie le groupement méthylénediphosphonate, PCHFP identifie le groupement monofluorométhylènediphosphonate, et PCF₂P identifie le groupement difluorométhylènediphosphonate.

### Schéma I :

### Pour R₁ : fonction alcool tertiaire, alcène, époxyde, et R₂ : PCP, PCHFP, PCF₂P :

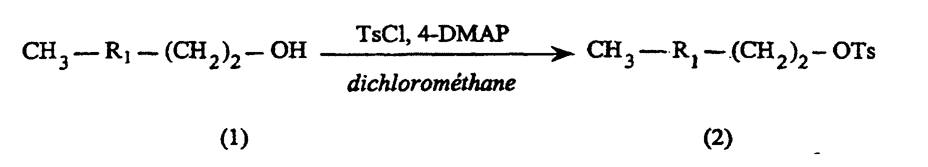

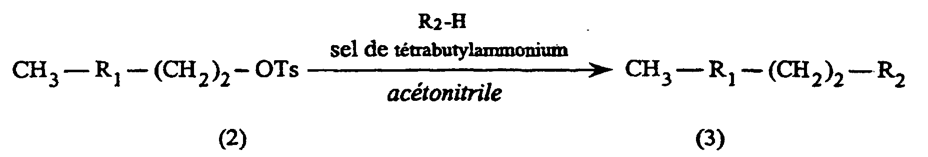

où Ts est le tosyle, TsCl est le chlorure de tosyle, 4-DMAP est la 4-diméthylaminopyridine.

Les sels de tétrabutylammonium du réactif R₂-H, utilisés en quantité au moins égale à 2 équivalents molaires sont, selon le groupement R₂ du composé à préparer:
- pour PCP: le tris(tétra-n-butylammonium) hydrogénométhylène-diphosphonate préparé à partir d'acide méthylène diphosphonique,
- pour PCF₂P: le tris(tétra-n-butylammonium) hydrogénodifluorométhylène-diphosphonate préparé à partir de tétrakis(triméthylsilyl)-difluorométhylènediphosphonate selon la procédure décrite par V. Jo DAVISSON et *al.* J. Org. Chem., 51, p 4768-4779, (1986),
- pour PCHFP : le tris(tétra-n-butylammonium) hydrogénomonofluorométhylènediphosphonate préparé à partir de tétrakis(triméthylsilyl)-monofluorométhylènediphosphonate selon la procédure décrite par J. NIESCHALK et *al.* (1996) Tetrahedron vol. 52 p165-176 et adaptée selon V. Jo DAVISSON *et al.* J. Org. Chem., 51, p 4768-4779, (1986).

Les alcools (1) sont des produits commercialement disponibles à l'exception de l'alcool correspondant à la fonction R1 époxyde qui peut être obtenu facilement (G. M. RUBOTTOM *et al.,* Org. Synth. Coll. Vol 7, p 282 (1990), Wiley) par époxydation de la fonction alcène selon:

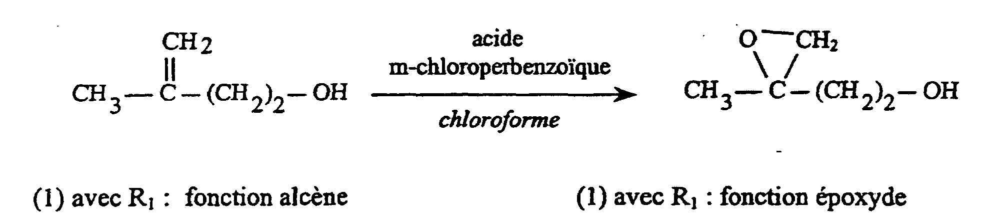

### Schéma II :

### Pour R₁ : fonction Halohydrine (X = CI, Br, I), et R₂: PCP, PCHFP, PCF₂P :

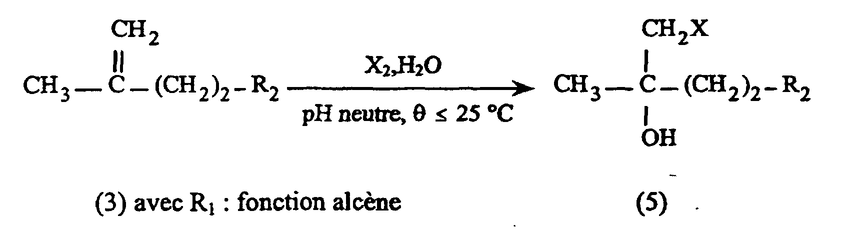

### Schéma III :

### Variante pour R₁ : fonction époxyde, et R₂ : PCP, PCHFP, PCF₂P:

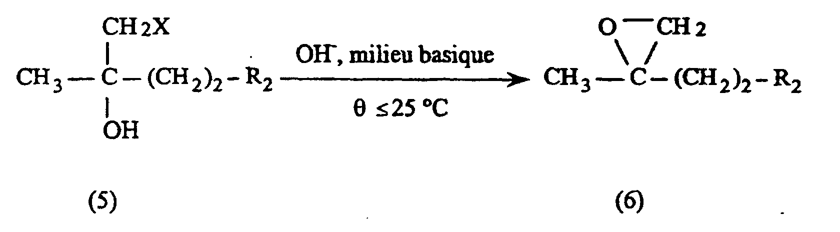

### Schéma IV :

### Pour R₁: fonction 1,2-diol, et R₂: PCP, PCHFP, PCF₂P:

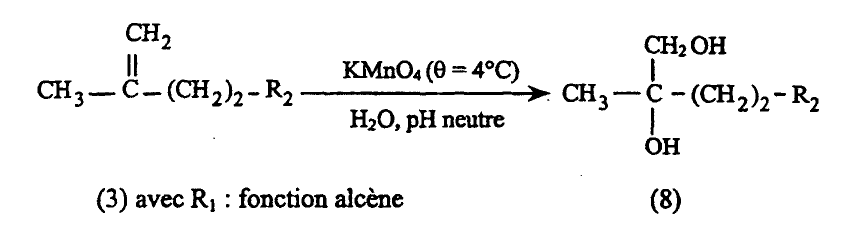

où KMnO₄ est le permanganate de potassium (en quantité inférieure ou égale à 1 équivalent molaire)

### Schéma V :

### Pour R₁: fonction aldéhyde, et R₂: PCP, PCHFP, PCF₂P:

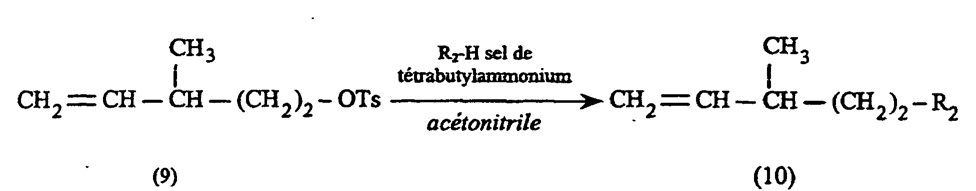

où R₂-H est utilisé en.quantité au moins égale à 2 équivalents molaires.

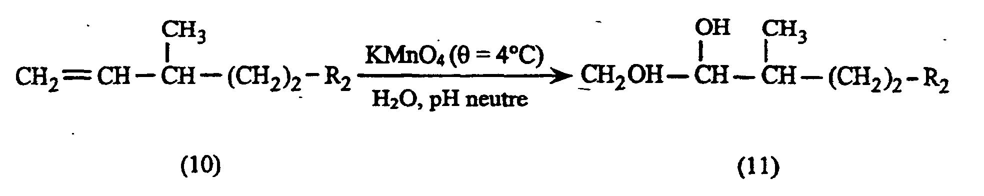

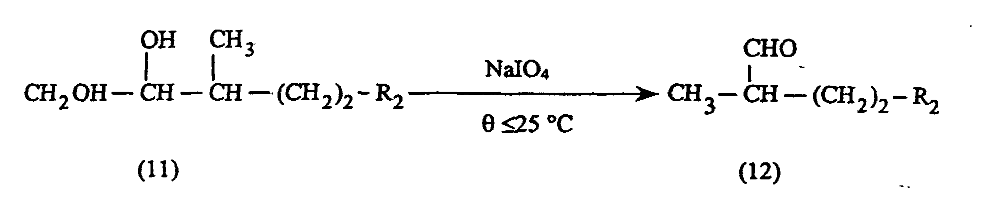

Le composé (9) peut être obtenu aisément sous forme alcool par synthèse de Grignard entre un organomagnésium d'alcényle et le formaldéhyde ou l'oxyde d'éthylène, par exemple en partant du 1-chloro-2-méthyl-3-butène.

### Schéma VI :

### Pour R₁: fonction α-hydroxyaldéhyde, et R₂: PCP, PCHFP, PCF₂P:

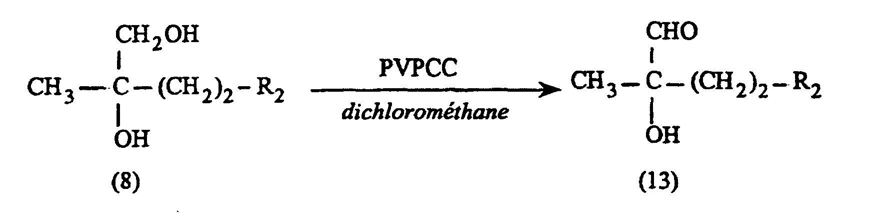

où PVPCC est le Poly[vinyl(pyridinium chlorocbromate)], comme indiqué par FRECHET J.M., WARNOCK J., et FARRALL J., J Org. Chem, vol 43, N° 13, p2618-21 (1978).

D'autres caractéristiques, buts et avantages de l'invention apparaissent à la lecture des exemples qui suivent et des figures annexées dans lesquelles:
- les figures 1 à 6 sont des graphes représentant les résultats obtenus à l'exemple 10,
- la figure 7 représente quatre graphes illustrant les résultats obtenus à l'exemple 11,
- la figure 8 représente un graphe illustrant les résultats obtenus à l'exemple 12,
- la figure 9 représente un graphe illustrant les résultats obtenus à l'exemple 13,
- les figures 10a, 10b et 10c illustrent les résultats obtenus à l'exemple 14.

### EXEMPLE 1 : Fabrication du 3-méthyl-3-butène-1-yl-méthylènediphosphonate (IPCP):

### Préparation du 3-méthyl-3-butène-1-yl -tosylate

Dans un réacteur en verre équipé pour la manipulation sous atmosphère inerte et soigneusement séché, sont introduits sous agitation magnétique (2,32 mmoles - 442mg) de chlorure de tosyle et (2,55 mmoles - 312 mg) de 4-(N,N-diméthylamino)pyridine dans 5 ml de dichlorométhane anhydre. A ce mélange, on ajoute lentement à l'aide d'une seringue et par l'intermédiaire d'un septum (2,32 mmoles - 200 mg) d'isopentènol en solution dans environ 1ml de dichlorométhane. La réaction est suivie par chromatographie sur couche mince de silice (gel de silice 60 F-254 - éluant : pentane/acétate d'éthyle 85/15 v/v - Rf(R-OTs) = 0,4 et Rf(TsCl) = 0,5). Après environ 3 heures d'agitation sous atmosphère d'azote on dilue le mélange réactionnel dans un grand volume d'hexane (environ 100ml) ce qui entraîne la formation immédiate d'un précipité blanc. Le mélange est ensuite filtré et le filtrat concentré par évaporation sous pression réduite. La solution est diluée avec un peu de diéthyl éther et filtrée à nouveau. Après évaporation du solvant, on obtient une huile jaunâtre. Le produit est purifié par chromatographie sur colonne préparative de silice (gel de silice 60 - éluant : pentane/acétate d'éthyle 85/15).(1,98 mmoles - 475 mg) de 3-méthyl-3-butène-1-yl -tosylate (85 % de rendement en produit isolé) sont ainsi obtenus. Le composé (huile incolore) est stocké à + 4°C en milieu anhydre.

### Préparation du tris(tetra-n-butylammonium) hydrogèno-méthylènediphosphonate :

On prépare une solution contenant (5,68 mmoles - 1g) d'acide méthylènediphosphonique dans environ 20 ml d'eau déionisée. A cette solution acide (pH 1.0), on ajoute goutte à goutte une solution aqueuse d'hydroxyde de tétra-n-butylammonium (Bu₄NOH) à 40% en masse jusqu'à obtenir une valeur de pH égale à 10.0. Après lyophilisation de la solution titrée, on obtient environ 5 g de sel de tétra-n-butylammonium (solide hygroscopique d'aspect huileux) que l'on dissout dans 10 ml d'acétonitrile anhydre. La solution saline est ensuite filtrée puis séchée par évaporations successives du solvant sous pression réduite. On obtient ainsi une solution de tris(tétra-n-butylammonium) hydrogèno-méthylènediphosphonate avec une pureté égale à 97 % (résultat déduit de l'analyse par chromatographie ionique - HPAEC). Le volume est ajusté avec de l'acétonitrile anhydre afin d'obtenir une concentration en sel comprise entre 0,5 et 1M. La solution est stockée à -20°C en milieu anhydre.

### Préparation du 3-méthyl-3-butène-1-yl-méthylènediphosphonate (isopentényl méthylènediphosphonate):

Dans un réacteur en verre soigneusement séché, on introduit sous atmosphère d'azote 2,5 ml d'une solution de tris(tétra-n-butylammonimm) hydrogèno-méthylènediphosphonate à 0,7 M (1,75 mmoles) dans l'acétonitrile anhydre. Le réacteur est refroidi par un bain de glace puis on ajoute sous agitation magnétique et à l'aide d'une seringue (0,70 mmoles - 168 mg) de 3-méthyl-3-butène-1-yl -tosylate en solution dans un minimum d'acétonitrile (0,5 - 1M). Après introduction du tosylate, le bain de glace est retiré puis la réaction est laissée sous agitation à température ambiante. L'avancement de la réaction est alors suivi par chromatographie ionique (HPAEC) sur colonne IonPac® AS11. Après environ 3 heures, le solvant est évaporé sous pression réduite et le milieu réactionnel redissout dans 3 ml d'un mélange eau /2-propanol 98/2 (v/v). La solution est passée sur une colonne contenant (19 mequiv - 4 g) de résine cationique DOWEX® 50-WX8-200 (forme NH₄⁺) puis éluée avec 10 ml du mélange eau (pH 9)/2-propanol 98/2 (v/v). Après lyophilisation, on recueille un solide blanc contenant le produit brut.

### Purification:

Le diphosphonate d'ammonium en excès et une faible proportion de sels inorganiques sont séparés du milieu réactionnel par co-précipitation en présence d'hydrogénocarbonate d'ammonium. On dissout le produit brut obtenu à l'étape précédente dans 4 ml d'hydrogénocarbonate d'ammonium 0,1 M que l'on transfère dans un tube à centrifugation de 25 ml. On traite alors la solution avec 10 ml d'un mélange acétonitrile/2-propanol 1/1 (v/v) en agitant vigoureusement le mélange (vortex) pendant quelques minutes jusqu'à formation d'un précipité. Le tube est ensuite centrifugé à 2000 tr/min à 10°C pendant 5 minutes. Le surnageant, dans lequel sont extraits les sels organiques, est conservé à +4°C. La procédure est renouvelée en redissolvant le précipité dans 3ml d'hydrogénocarbonate d'ammonium 0,1 M auxquels on ajoute 7 ml du mélange acétonitrile/2-propanol. Après élimination du solvant de l'ensemble des surnageants dans un évaporateur rotatif, on obtient un liquide huileux que l'on conserve à +4°C.

Le tosylate d'ammonium est en majeure partie séparé du milieu réactionnel par extraction avec le solvant chloroforme/méthanol 1/1 (v/v). Le liquide huileux de l'étape précédente est dissout dans 4 ml d'eau déionisé à pH 9 et traité avec 1ml de ce solvant par une procédure classique d'extraction répétée 3 fois. On élimine ensuite de la phase aqueuse les traces de solvant par évaporation sous pression réduite à 30 °C. La solution est stockée à -20°C.

Le produit est purifié ultérieurement selon les besoins par chromatographie d'échange d'anions sur cartouches Sep-Pak Accell Plus QMA (Waters®) de 360 mg à 10 grammes éluées successivement par des solutions aqueuses d'hydrogénocarbonate d'ammonium respectivement de 20 mM, 40 mM, 100 mM, puis 200 mM avec suivi chromatographique (HPAEC) des fractions éluées. Les fractions correspondant au produit purifié sont regroupées puis lyophilisées. Pour la mise en oeuvre de tests biologiques les solutions aqueuses du produit sont stérilisées par filtration sur filtre de 0,2 µm et stockées à -20 °C. Dans le cas de tests réalisés in vivo, les solutions sont préalablement passées sur une colonne de résine cationique DOWEX® 50-WX8-200 (forme Na⁺) éluée par deux volumes de colonne d'eau déionisée.

Analyse du sel d'ammonium par spectrométrie de masse à ionisation dite par "électrospray" (mode négatif):
ESI-MS: m/z = 243 [M-H]⁻ espèce pseudomoléculaire
ESI-MS/MS de l'ion [M-H]⁻: m/z = 225 (perte d'H₂O); m/z = 157 (pyrophosphonate)

### EXEMPLE 2 : Fabrication du 3-(bromométhyl)-3-butanol-1-yl-méthylènediphosphonate (BrHPCP):

0,34 mmoles (100 mg) de 3-méthyl-3-butène-1-yl-méthylènediphosphonate (sel d'ammonium) en solution dans 2 ml d'eau déionisée de pH neutre sont traitées sous une hotte aspirante par 1,9 ml d'une solution aqueuse saturée (0,18 M) d'eau de brome (1 équivalent - 0,34 mmoles de brome). L'eau de brome est ajoutée progressivement et de préférence sur une solution froide du sel d'ammonium en agitant périodiquement jusqu'à décoloration de l'eau de brome. Dans le cas où le brome est ajouté en léger excès (coloration jaune persistante), la solution est transférée dans un ballon en verre puis placée quelques minutes sous pression réduite (évaporateur rotatif) à une température de 30 °C jusqu'à disparition de la coloration. Le produit 3-(bromométhyl)-3-butanol-1-yl-méthylènediphosphonate est généré quantitativement (0,33 mmoles - 130 mg)-résultat déduit de l'analyse par chromatographie ionique - HPAEC. La solution est ensuite traitée comme dans l'exemple 1 pour la mise en oeuvre de tests biologiques et stockée à -20°C.

Analyse du sel d'ammonium par spectrométrie de masse à ionisation dite par "électrospray" (mode négatif) :
ESI-MS: m/z = 339, 341 isotopes naturels du brome présent dans l'espèce pseudomoléculaire [M-H]⁻
ESI-MS/MS de l'ion [M-H]⁻: m/z = 259 (réarrangement intramoléculaire)

### EXEMPLE 3 : Fabrication du 3-(iodométhyl)-3-butanol-1-yl-méthylènediphosphonate (IHPCP):

### Préparation de l'eau iodée:

Une solution d'eau iodée de l'ordre de 0,5 à 1 mM est préparée par sonication prolongée (15 minutes environ) de quelques cristaux d'iode dans une solution d'eau déionisée et filtration. Pour des essais portant sur de plus grandes quantités, des solutions plus concentrées en iode peuvent être obtenues en rajoutant une faible proportion d'alcool à la solution aqueuse initiale. L'eau iodée est ensuite titrée par le thiosulfate de sodium avec de l'empois d'amidon comme indicateur coloré.

### Préparation du 3-(iodométhyl)-3-butanol-1-yl-méthylènediphosphonate :

5 µmoles (1 ml d'une solution à 5 mM) de 3-méthyl-3-butène-1-yl-méthylènediphosphonate préparé selon l'exemple 1 sous forme de sel d'ammonium en milieu aqueux ou hydroalcoolique de pH neutre sont traitée à température ambiante par ajout de 1 équivalent d'iode en solution aqueuse (5 ml d'eau iodée à 1 mM). La solution est placée 30 minutes à température ambiante, puis 30 minutes à +4 °C en effectuant périodiquement une agitation vigoureuse. Après décoloration de l'eau iodée, le produit 3-(iodométhyl)-3-butanol-1-yl-méthylènediphosphonate est généré quantitativement. Pour la mise en oeuvre de tests biologiques, la solution est préalablement concentrée par lyophilisation et traitée comme dans l'exemple 1.

### EXEMPLE 4 : Fabrication du 3,4-époxy-3-méthyl-1-butyl-méthylènediphosphonate (Epox PCP) :

On traite à température ambiante, 1 ml d'une solution aqueuse contenant (2 mg - 5,1 µmoles) de 3-(bromométhyl)-3-butanol-1-yl-méthylènediphosphonate (sel d'ammonium) préparé selon l'exemple 2, avec 0,5 ml d'une solution molaire d'ammoniaque. La solution est maintenue sous agitation pendant quelques minutes puis lyophilisée pour éliminer l'ammoniaque. Le résidu sec obtenu après lyophilisation est redissout dans 1 ml d'eau déionisée et purifié par chromatographie d'échange d'anions sur cartouches Sep-Pak Accell Plus QMA (Waters®) de 360 mg comme décrit dans l'exemple 1.

Analyse du sel d'ammonium par spectrométrie de masse à ionisation dite par "électrospray" (mode négatif):
ESI-MS : m/z = 259 [M-H]⁻ espèce pseudomoléculaire
ESI-MS/MS de l'ion [M-H]⁻ : m/z = 241 (perte d'H₂0) ; m/z = 157 (pyrophosphonate)

### EXEMPLE 5 : Fabrication du 3-méthyl-3-butanol-1-yl-méthylènediphosphonate (tButOHPCP) :

Selon une procédure analogue à celle décrite dans l'exemple 1, on prépare dans une première étape le 3-méthyl-3-butanol-1-yl-tosylate à partir de 3-méthyl-1,3-butanediol. Le 3-méthyl-3-butanol-1-yl-méthylènediphosphonate est obtenu en faisant réagir 0,5 mmole de tosylate et 1 mmole de tris(tetra-n-butylammonium) hydrogèno-méthylènediphosphonate à température ambiante pendant 24 heures. La procédure de purification est identique à celle décrite dans l'exemple 1.

Analyse du sel d'ammonium par spectrométrie de masse à ionisation dite par "électrospray" (mode négatif) :
ESI-MS: m/z =261 [M-H]⁻ espèce pseudomoléculaire
ESI-MS/MS de l'ion [M-H]⁻ : m/z = 243 (perte d'H₂0) ; m/z = 157 (pyrophosphonate)

### EXEMPLE 6 : Fabrication du 3-méthyl-3,4-butanediol-1-yl-méthylènediphosphonate (Diol PCP) :

Dans une fiole en verre, on introduit 1 ml d'une solution aqueuse de pH neutre du sel d'ammonium du 3-méthyl-3-butène-1-yl-méthylènediphosphonate (3,4 µmoles - 1 mg) - préparé selon l'exemple 1 - A cette solution sont ajoutés en plusieurs fractions, 680 µl d'une solution froide de permanganate de potassium à 5 mM (1 équivalent - 3,4 µmoles) en agitant périodiquement la solution que l'on place en chambre froide (+4°C). Après environ 40 minutes de réaction pendant lesquelles il s'est formé un précipité marron de dioxyde de manganèse, on ajoute quelques microlitres d'une solution aqueuse saturée d'isopentènol. Le dioxyde de manganèse est séparé du mélange réactionnel par centrifugation puis filtration. Le filtrat est purifié par chromatographie d'échange d'anions sur cartouches Sep-Pak Accell Plus QMA (Waters®) de 360 mg comme décrit dans l'exemple 1.

### EXEMPLE 7 : Fabrication du 3-méthyl-3-butène-1-yl-difluorométhylènediphosphonate (IPCF₂P):

Ce produit est préparé comme décrit dans l'exemple 1, en faisant réagir dans l'acétonitrile anhydre, 0,5 mmole de 3-méthyl-3-butène-1-yl-tosylate avec (3 équivalents - 1,5 mmoles) du sel de tris(tetra-n-butylammonium) hydrogèno-difluorométhylènediphosphonate préparé selon le protocole décrit par V.Jo Davisson *et al.* J. Org. Chem., 1986, 51 p 4768-4779.

Analyse du sel d'ammonium par spectrométrie de masse à ionisation dite par "électrospray" (mode négatif):
ESI-MS: m/z = 279 [M-H]⁻ espèce pseudomoléculaire
ESI-MS/MS de l'ion [M-H]⁻: m/z = 261 (perte d'H₂O); m/z = 193 (pyrophosphonate)

### EXEMPLE 8 : Fabrication du 3-(bromométhyl)-3-butanol-1-yl-difluorométhylènediphosphonate (BrHPCF₂P):

Ce produit est obtenu par réaction du 3-méthyl-3-butène-1-yl-difluorométhylènediphosphonate (préparé selon l'exemple 7) avec l'eau de brome en suivant la procédure décrite dans l'exemple 2.

Analyse du sel d'ammonium par spectrométrie de masse à ionisation dite par "électrospray" (mode négatif):
ESI-MS: m/z = 375, 377 isotopes naturels du brome présent dans l'espèce pseudomoléculaire [M-H]⁻
ESI-MS/MS de l'ion [M-H]⁻: m/z = 295 (réarrangement intramoléculaire)

### EXEMPLE 9 : Fabrication du 3,4-époxy-3-méthyl-1-butyl-difluorométhylènediphosphonate(Epox PCF₂P):

Ce produit est obtenu par traitement en milieu basique du 3-(bromométhyl)-3-butanol-1-yl-difluorométhylènediphosphonate (préparé selon l'exemple 8) en suivant la procédure décrite dans l'exemple 4.

Analyse du sel d'ammonium par spectrométrie de masse à ionisation dite par "électrospray" (mode négatif):
ESI-MS: m/z = 295 [M-H]⁻ espèce pseudomoléculaire
ESI-MS/MS de l'ion [M-H]⁻: m/z = 277 (perte d'H₂0) ; m/z = 193 (difluorométhylènediphosphonate)

### EXEMPLE 10 : Mesure de l'activité cytotoxique d'un clone Tγ9δ2 activé par 80nM de BrHPP, ou non activé :

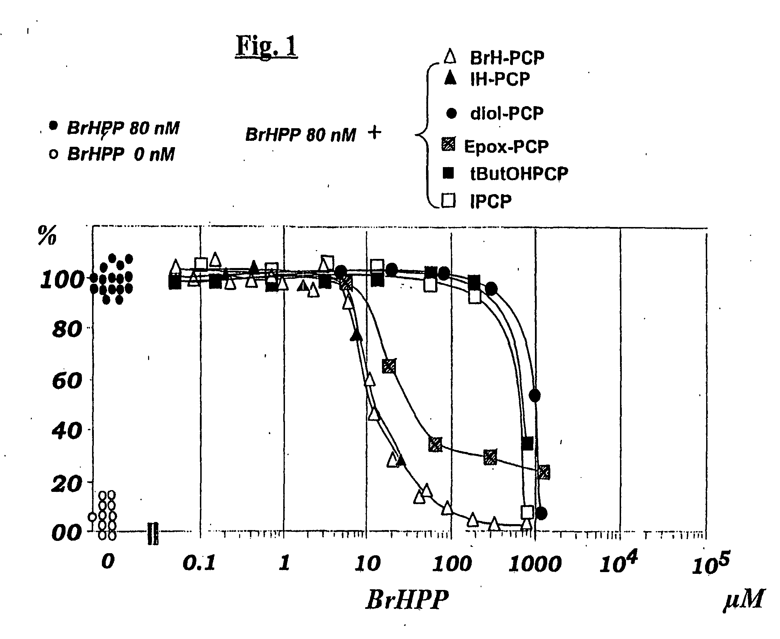

On compare l'activité cytotoxique spécifique d'un clone de lymphocytes Tγ9δ2 mesurée selon le test de cytotoxicité induite, cette activité étant stimulée avec 80nM de l'antigène 3-(bromométhyl)-3-butanol-1-yl-diphosphate (BrHPP) (petits points noirs en haut à gauche de la figure 1), et considérée comme la réponse de référence (100%), par rapport à celle d'une culture de clones non stimulés (0%) (petits points blancs figure 1).

Les courbes de la figure 1 représentent le pourcentage de réponse résiduelle (test de cytotoxicité induite) obtenu dans des cultures stimulées par 80nM de BrHPP en présence de différentes concentrations (en abscisses) des composés selon l'invention, à savoir BrHPCHFP (triangles blancs), IHPCP (triangles noirs), Diol PCP (ronds noirs), Epox PCP (carrés gris et croix), tButOHPCP (carrés noirs), et IPCP (carrés blancs), tels qu'obtenus aux exemples précédents.

On constate que l'ajout de concentrations croissantes de ces composés inhibe jusque 100% de la réponse de référence.

Les essais effectués comme indiqué ci-dessus sur différents composés selon l'invention permettent de définir leurs concentrations IC50, exprimées en micromolaire dans le tableau suivant, conduisant à l'inhibition de 50% de la réponse de référence des lymphocytes stimulés par 80nM du composé BrHPP selon le test de cytotoxicité induite.

| **Composé** | **µM** |
|---|---|
| I PCP | 700 |
| tButOH PCP | 1000 |
| Epox PCP | 30 |
| BrH PCP | 15 |
| IH PCP | 15 |
| I PCF₂P | 1000 |
| Epox PCF₂P | 300 |
| BrH PCF₂P | 150 |

D'autres essais semblables ont aussi été effectués avec les composés analogues monofluorés (où le groupement R₂ est le monofluorométhylènediphosphonate) BrHPCHFP et Epox PCHFP. Ces composés sont bioactifs (c'est-à-dire inhibent sélectivement les lymphocytes Tγ9δ2), avec une bioactivité de 30µM pour BrHPCHFP et de 50µM pour Epox PCHFP, pour une concentration de BrHPP égale à 150µM.

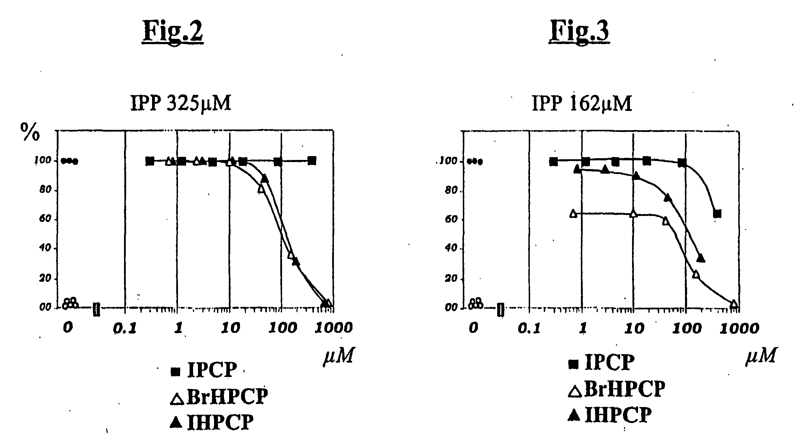

Les figures 2 et 3 sont des graphes semblables à la figure 1 obtenus en remplaçant l'antigène BrHPP par l'antigène IPP (isopenténylpyrophosphate) à 325µM ou, respectivement, à 162 µM. Les composés selon l'invention utilisés étaient dans ces exemples IPCP (carrés noirs), BrHPCP (triangles blancs) et IHPCP (triangles noirs). Comme on le voit, l'inhibition par les composés selon l'invention ne dépend pas de l'antigène utilisé pour stimuler les lymphocytes Tγ9δ2.

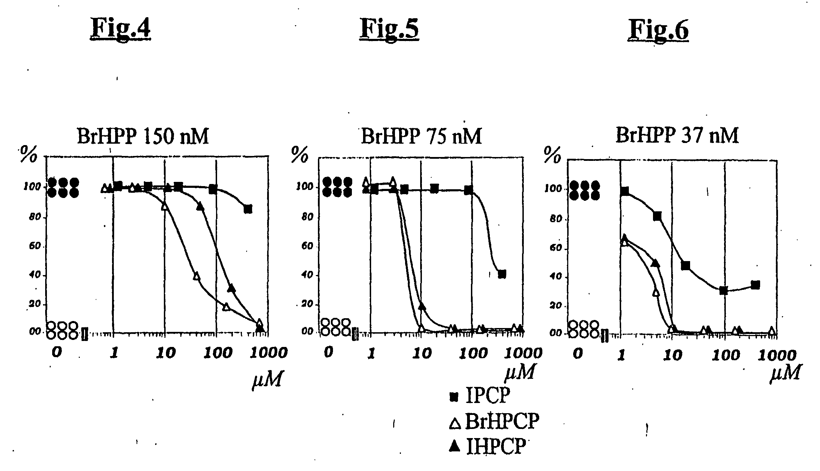

Les figures 4 à 6 illustrent les résultats obtenus avec ces mêmes trois composés selon l'invention mais en utilisant comme antigène stimulant les lymphocytes Tγ9δ2, le composé BrHPP à une concentration variant, respectivement, de 150µM, 75µM et 37µM. Comme on le voit, les composés selon l'invention produisent l'inhibition des lymphocytes dans tous les cas, mais à des concentrations qui varient dans le même sens que les concentrations d'antigène de stimulation utilisées. Autrement dit, plus la concentration d'antigène stimulant est forte, plus la concentration de composé selon l'invention nécessaire pour inhiber les lymphocytes doit aussi être forte.

### EXEMPLE 11 : Mesure de l'activité inhibitrice et de son caractère réversible, par le test de cytotoxicité induite et le test de relargage de TNF :

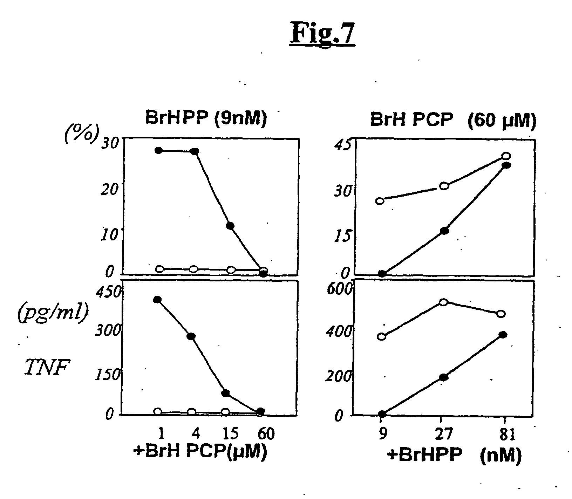

La figure 7 représente quatre graphes illustrant l'inhibition par le composé selon l'invention BrHPCP et la restauration de l'activité antigénique stimulante du BrHPP.

Les deux graphes de gauche sont obtenus en stimulant les lymphocytes Tγ9δ2 comme à l'exemple 12 en ajoutant tout d'abord 9nM de l'antigène BrHPP dans le milieu de culture, puis en ajoutant des concentrations croissantes (en abscisses) du composé selon l'invention BrHPCP. Les deux graphes de droite sont obtenus en incorporant tout d'abord 60µM du composé selon l'invention BrHPCP dans le milieu de culture au contact des lymphocytes Tγ9δ2 puis en ajoutant des concentrations croissantes (en abscisses) du composé antigénique stimulant BrHPP. Les valeurs obtenues sont représentée par des ronds noirs. Les ronds blancs donnent les valeurs obtenues en l'absence du composé initial (BrHPP sur les graphes de gauche, BrHPCP sur les graphes de droite). Les graphes du haut fournissent le pourcentage de réponse-résiduelle dans les cultures (test de cytotoxicité induite). Les graphes du bas fournissent la concentration de TNF relarguée en pg/ml.

Comme on le voit, le composé selon l'invention BrHPCP inhibe la stimulation par BrHPP, mais cette inhibition est réversible dans la mesure où, après inhibition par le composé selon l'invention BrHPCP, la stimulation est restaurée par ajout de BrHPP.

Ce caractère réversible de l'inhibition des lymphocytes Tγ9δ2 par les composés selon l'invention est important du point de vue thérapeutique. En effet, suite à un traitement d'une activation massive à caractère pathogène des lymphocytes Tγ9δ2 grâce à un composé selon l'invention (par exemple lors d'un accès de paludisme ou sur une tumeur), le système immunitaire du patient n'est pas pour autant définitivement dégradé et ensuite, peut être rapidement restauré.

### EXEMPLE 12 : BrHPCP n'est pas inhibiteur des lymphocytes Tγ8δ3:

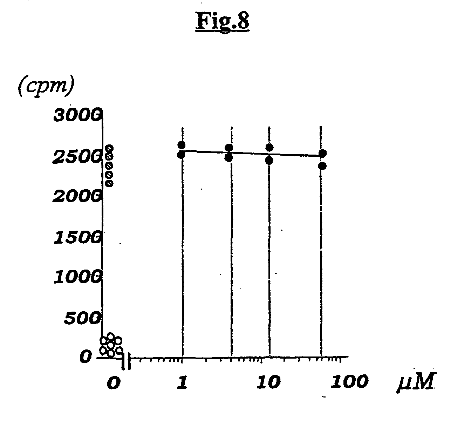

Un test de cytotoxicité induite est réalisé comme dans l'exemple 12, mais avec un clone de lymphocyte Tγ8δ3 stimulé par un antigène traditionnel de ces lymphocytes (ronds noirs), et en présence du composé selon l'invention BrHPCP en concentrations croissantes dans le milieu de culture (carrés noirs sur la figure 8). Comme on le voit figure 8, le composé selon l'invention n'inhibe pas les lymphocytes Tγ8δ3. Il est donc un inhibiteur spécifique des lymphocytes Tγ9δ2.

### EXEMPLE 13:

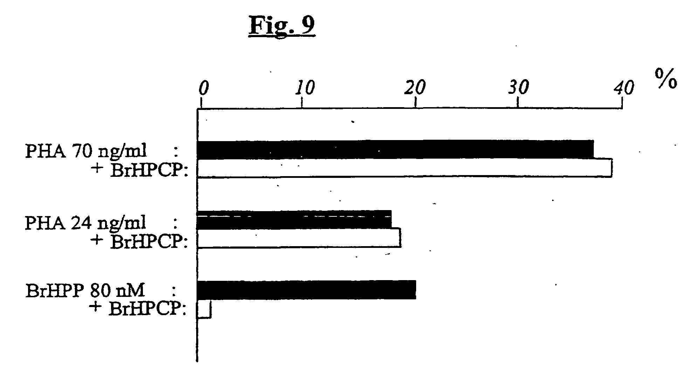

Dans cet exemple, on réalise un test de cytotoxicité induite sur des cellules cibles P815 par un clone de lymphocytes Tγ9δ2 stimulé soit par la phytohémaglutinine A (PHA), qui est un stimulant non spécifique et non phosphaté des lymphocytes Tγ9δ2, à 70 ng/ml et à 24 ng/ml, soit par l'antigène BrHPP à 80nM. Le stimulant est utilisé seul (barres blanches figure 9) soit en présence du composé inhibiteur selon l'invention BrHPCP à 70µM (barres noires figure 9).

Comme on le voit, le composé selon l'invention n'inhibe pas les lymphocytes activés par le stimulant non spécifique PHA. Il n'inhibe donc les lymphocytes Tγ9δ2 que s'ils ont été préalablement stimulés de façon spécifique par un antigène diphosphaté (phosphoantigène) tel que BrHPP.

### EXEMPLE 14:

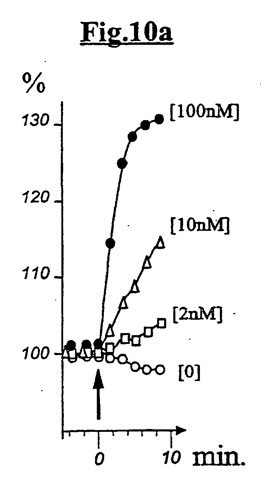

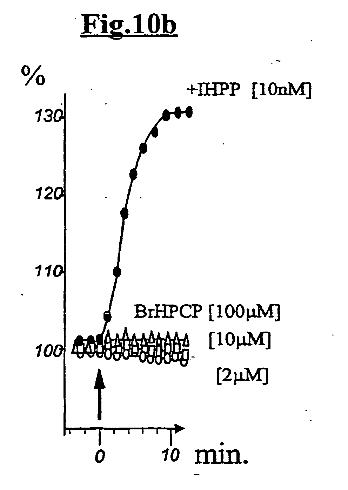

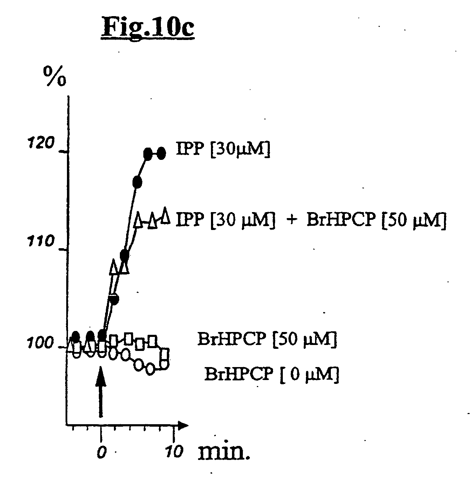

Un million de lymphocytes Tγ9δ2 sont déposés dans un puits de 10 µl d'un microphysiomètre (appareil CYTOSENSOR ® commercialisé par MOLECULAR DEVICES, USA). Leur vitesse de métabolisme donnée par l'appareil est mesurée toutes les 30 secondes. On ajoute dans le puits une composition comprenant soit l'antigène BrHPP à 0, 2, 10 et 100nM (figure 10a), soit BrHPCP - à 2, 10, 100µM (ronds, carrés et triangles blancs figure 10b), soit l'antigène IHPP (3-(iodométhyl)-3-butanol-1-yl-diphosphate) à 10nM (ronds noirs figure 10b), en tant que référence, soit une composition de contrôle inactive (ronds blancs figure 10c), soit BrHPCP à 50µM seul (carrés blancs figure 10c), soit IPP (isopenténylpyrophosphate) seul à 30µM (ronds noirs figure 10c), soit IPP à 30µM et BrHPCP à 50µM (triangles blancs figure 10c).

L'instant d'adjonction des compositions est représenté par la flèche sur les figures 10a, 10b, 10c.

Comme on le voit, comparativement à la réponse détectée dès l'addition des antigènes, les composés selon l'invention n'induisent pas de réponse (figure 10b) et diminuent la réponse aux phosphoantigènes (figure 10c) des lymphocytes Tγ9δ2.

Le suivi de l'expérience de la figure 10c sur une durée plus grande révèle également que la durée pendant laquelle les lymphocytes Tγ9δ2 sont activés est aussi diminuée en présence des composés selon l'invention.

## Revendications

1. Composés de formule :
CH₃―R₁―(CH₂)₂―R₂ (I)
où R₁ est choisi parmi les fonctions suivantes : 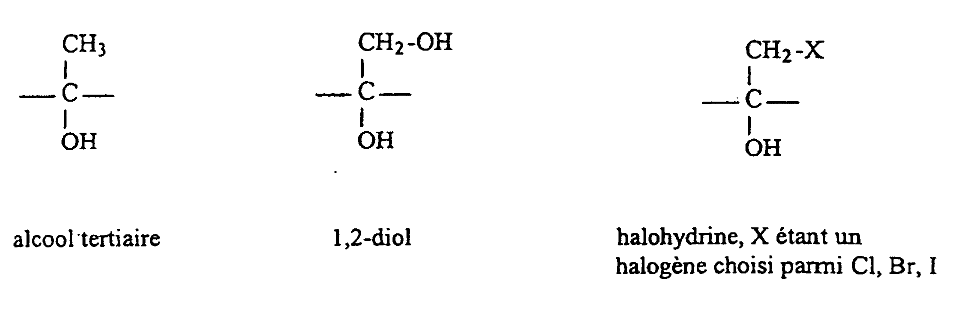 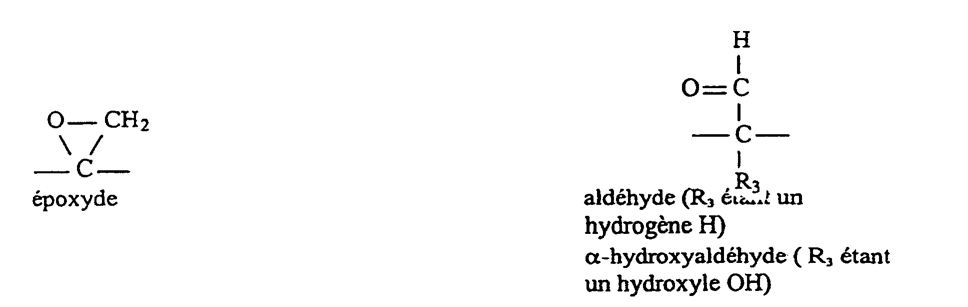 et R₂ est choisi parmi l'un des groupements suivants : 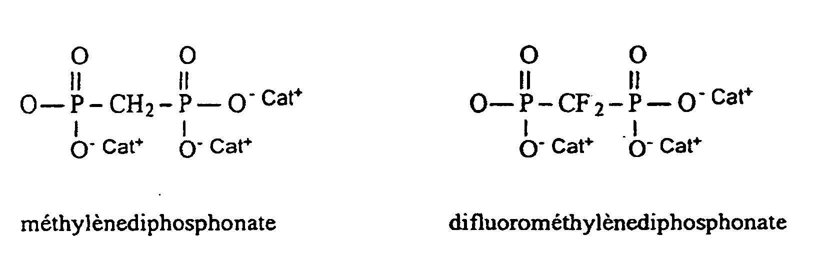 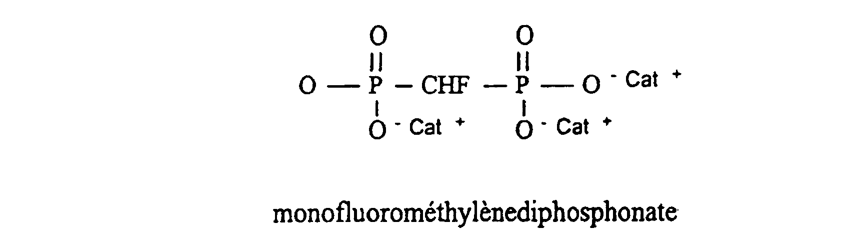 où Cat+ représente un (ou des) cation(s) organique(s) ou minéral(aux) (y compris le proton) identiques ou différents dans le même composé.

2. Composés de formule :
CH₃―R₁―(CH₂)₂―R₂ (I)
où R₁ est choisi parmi les fonctions suivantes : 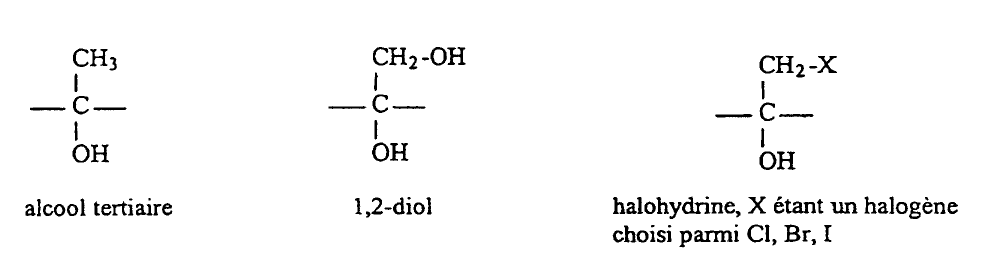 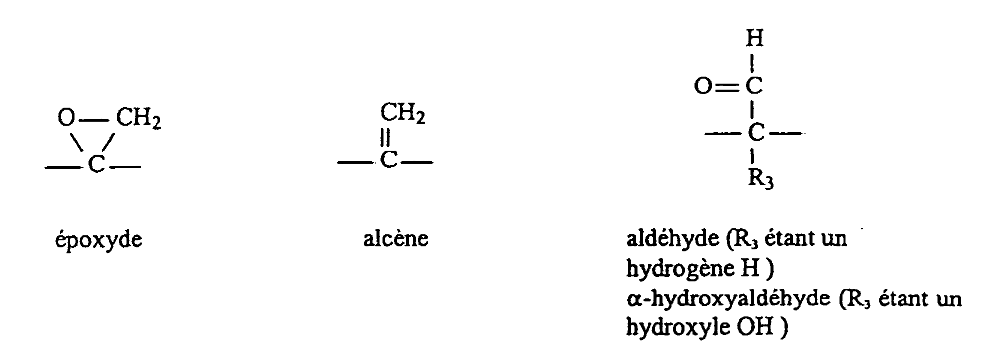 et R₂ est choisi parmi l'un des groupements suivants : 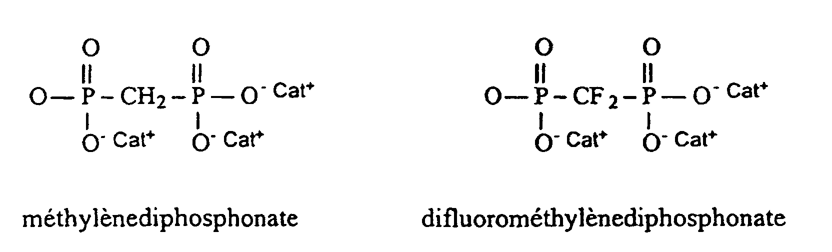 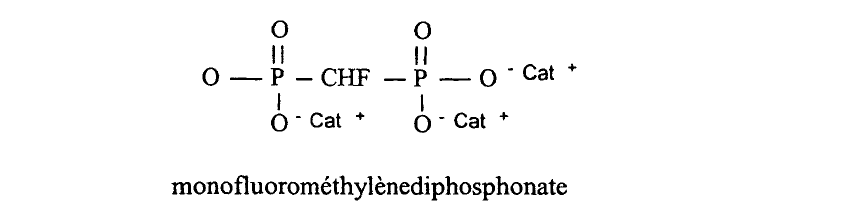 où Cat+ représente un (ou des) cation(s) organique(s) ou minéral(aux) (y compris le proton) identiques ou différents dans le même composé,
pour leurs utilisations à titre de substances thérapeutiquement actives.

3. Composés selon la revendication 1 pour leur utilisation à titre de substances thérapeutiquement actives.

4. Composés selon l'une des revendications 1 ou 2 pour leur utilisation dans une composition thérapeutique destinée à être administrée à un primate en vue du traitement préventif ou curatif d'une pathologie impliquant l'activation des lymphocytes Tγ9δ2.

5. Composition thérapeutique ou de diagnostic, **caractérisée en ce qu'**elle comporte au moins un composé selon l'une des revendications 1 ou 2.

6. Composition selon la revendication 5, **caractérisée en ce qu'**elle comprend une quantité d'au moins un composé selon l'une des revendications 1 ou 2 apte à être administrée à un primate.

7. Composition selon la revendication 5, **caractérisée en ce qu'**elle comprend une quantité d'au moins un composé selon l'une des revendications 1 ou 2 apte à être administrée à un primate au contact du sang périphérique ou par voie topique.

8. Procédé de fabrication d'une composition ayant la propriété d'inhiber sélectivement les lymphocytes Tγ9δ2 dans lequel on incorpore dans la composition au moins un composé selon l'une des revendications 1 ou 2 en une quantité apte à inhiber sélectivement les lymphocytes Tγ9δ2 lorsque ladite composition est placée au contact d'un milieu biologique contenant des lymphocytes Tγ9δ2.

9. Procédé de fabrication d'une composition thérapeutique destinée au traitement préventif ou curatif d'une pathologie induisant une activation des lymphocytes Tγ9δ2 dans lequel on utilise au moins un composé selon l'une des revendications 1 ou 2.

10. Procédé de fabrication d'une composition thérapeutique destinée à être administrée à un primate pour le traitement préventif ou curatif d'une pathologie induisant une activation des lymphocytes Tγ9δ2 dans lequel on utilise au moins un composé selon l'une des revendications 1 ou 2.

11. Procédé de fabrication d'une composition thérapeutique destinée à être administrée à un primate pour le traitement préventif ou curatif d'une pathologie induisant une activation des lymphocytes Tγ9δ2 dans lequel on utilise au moins un composé selon l'une des revendications 1 ou 2 au contact du sang périphérique ou par voie topique.

12. Procédé de fabrication d'une composition thérapeutique destinée à être administrée à un primate pour le traitement préventif ou curatif des parasitoses appartenant au groupe formé de la malaria, de la leishmaniose viscérale, et de la toxoplasmose, dans lequel on utilise au moins un composé selon l'une des revendications 1 ou 2.

13. Procédé de fabrication d'une composition thérapeutique destinée à être administrée à un primate pour le traitement préventif ou curatif d'une maladie auto-immune impliquant une activation des lymphocytes Tγ9δ2 dans lequel on utilise au moins un composé selon l'une des revendications 1 ou 2.

14. Procédé de fabrication d'une composition thérapeutique destinée à être administrée à un primate pour le traitement préventif ou curatif d'une maladie auto-immune impliquant une activation des lymphocytes Tγ9δ2 choisie parmi la sclérose en plaques et la maladie de Behçet, dans lequel on utilise au moins un composé selon l'une des revendications 1 ou 2.

15. Procédé de fabrication d'une composition thérapeutique destinée à être administrée à un primate pour le traitement préventif ou curatif d'une pathologie bactérienne appartenant au groupe formé de la brucellose, de la tularémie, des salmonelloses, des tuberculoses, et de l'ehrlichiose, dans lequel on utilise au moins un composé selon l'une des revendications 1 ou 2.

16. Procédé d'inhibition sélective de lymphocytes Tγ9δ2 dans un milieu extracorporel dans lequel on met les lymphocytes Tγ9δ2 au contact, dans ledit milieu extracorporel, avec au moins un composé de formule:
CH₃―R₁―(CH₂)₂―R₂ (I)
où R₁ est choisi parmi les fonctions suivantes : 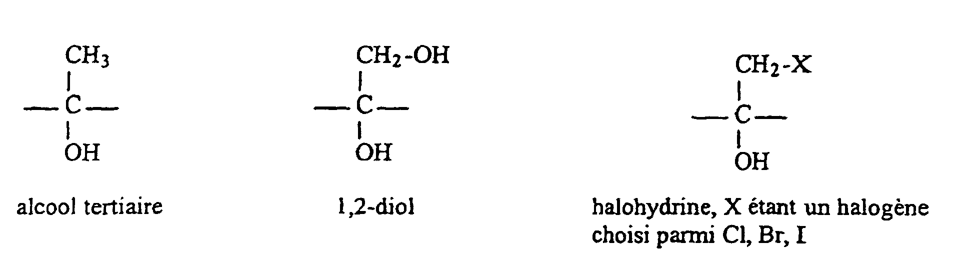 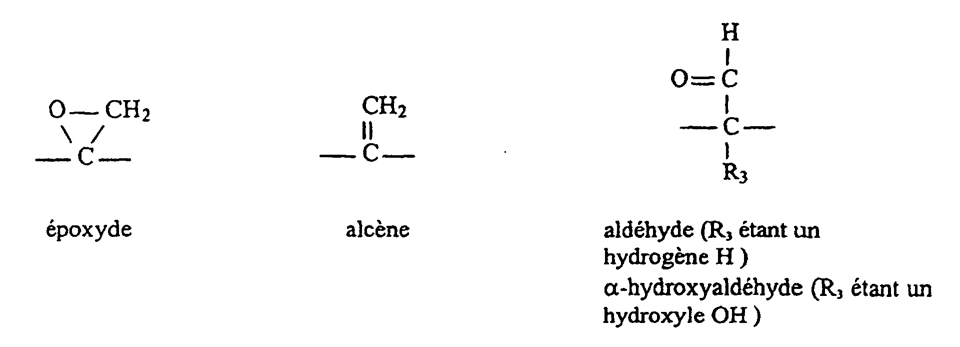 et R₂ est choisi parmi l'un des groupements suivants : 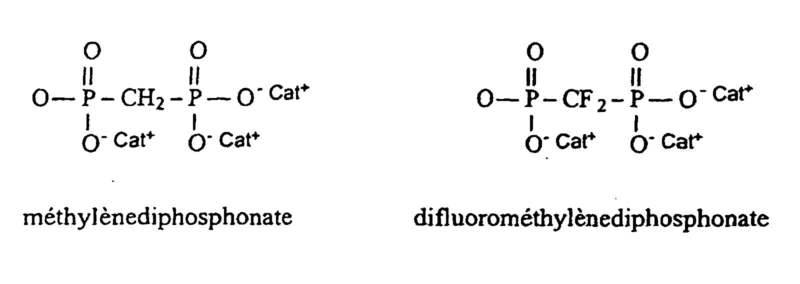 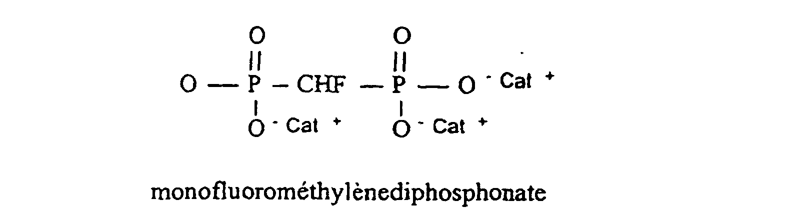 où Cat+ représente un (ou des) cation(s) organique(s) ou minéral(aux) (y compris le proton) identiques ou différents dans le même composé.

17. Procédé selon la revendication 16, **caractérisé en ce qu'**on utilise au moins un composé selon la formule (I) à une concentration dans le milieu qui procure une inhibition de la prolifération polyclonale des lymphocytes Tγ9δ2.

## Patentansprüche

1. Verbindungen der Formel:
CH₃-R₁-(CH₂)₂-R₂ (I)
wobei R₁ aus den folgenden Funktionsgruppen ausgewählt ist: 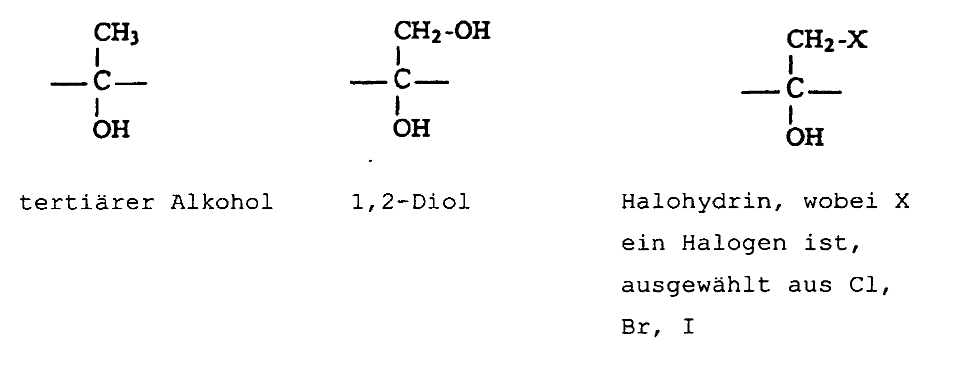 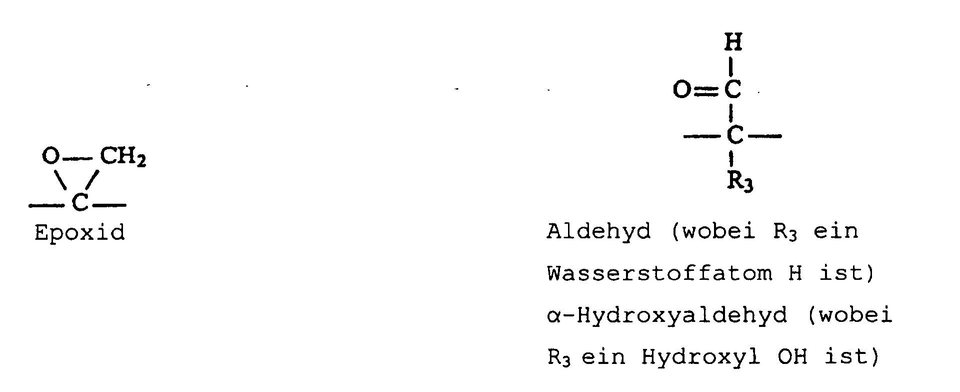 und R₂ aus einer der folgenden Gruppierungen ausgewählt ist: 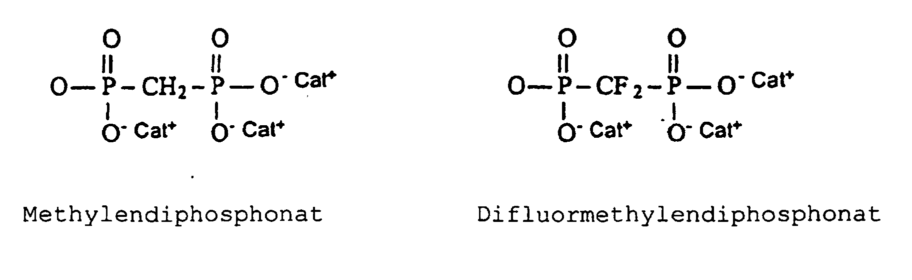 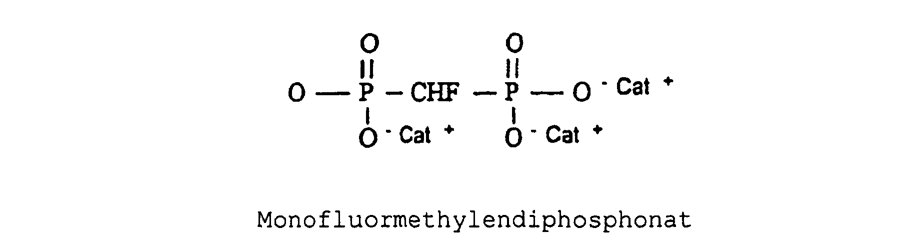 wobei Cat+ ein oder mehrere organische(s) oder mineralische(s) Kation(en) repräsentiert (einschließlich des Protons), die mit denen in derselben Verbindung identisch sind oder sich von ihnen unterscheiden.

2. Verbindungen der Formel:
CH₃-R₁-(CH₂)₂-R₂ (I)
wobei R₁ aus den folgenden Funktionsgruppen ausgewählt ist: 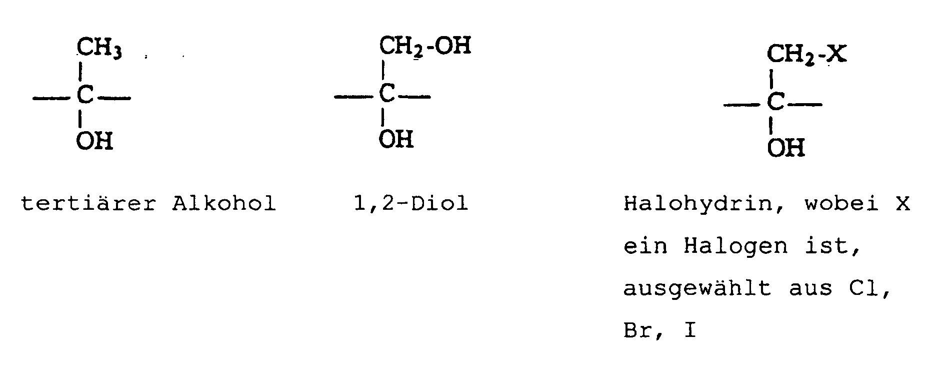 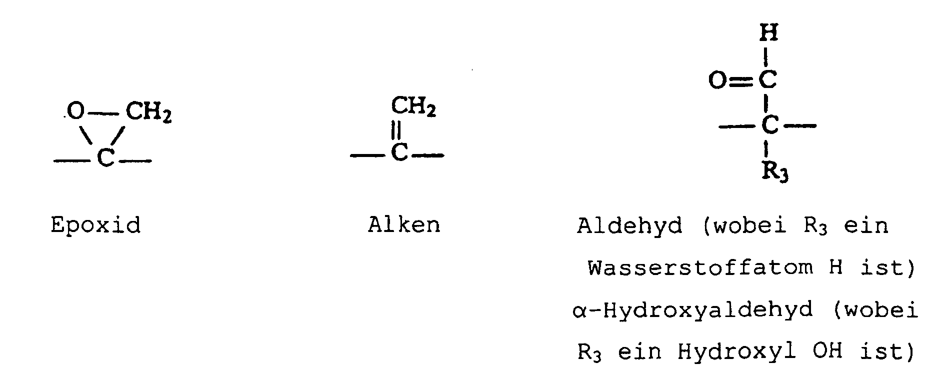 und R₂ ausgewählt ist aus einer der folgenden Gruppierungen: 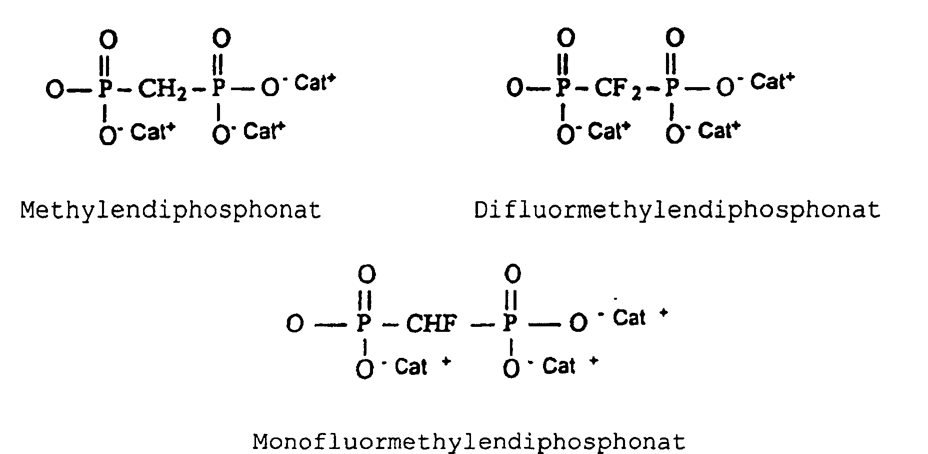 wobei Cat+ ein oder mehrere organische(s) oder mineralische(s) Kation(en) repräsentiert (einschließlich des Protons), die mit denen in derselben Verbindung identisch sind oder sich von ihnen unterscheiden, für deren Verwendung als therapeutisch aktive Substanzen.

3. Verbindungen nach Anspruch 1 zur Verwendung als therapeutisch aktive Substanzen.

4. Verbindungen nach Anspruch 1 oder 2 zur Verwendung in einer therapeutischen Zusammensetzung, die einem Primaten zur präventiven oder kurativen Behandlung einer Pathologie verabreicht werden soll, die die Aktivierung von Tγ9δ2-Lymphozyten impliziert.

5. Zusammensetzung für Therapie oder Diagnose, **dadurch gekennzeichnet, dass** sie wenigstens eine Verbindung nach Anspruch 1 oder 2 umfasst.

6. Zusammensetzung nach Anspruch 5, **dadurch gekennzeichnet, dass** sie eine Menge von wenigstens einer Verbindung nach Anspruch 1 oder 2 umfasst, die geeignet ist, um sie einem Primaten zu verabreichen.

7. Zusammensetzung nach Anspruch 5, **dadurch gekennzeichnet, dass** sie eine Menge von wenigstens einer Verbindung nach Anspruch 1 oder 2 umfasst, die geeignet ist, um sie einem Primaten in Kontakt mit peripherem Blut oder auf topischem Wege zu verabreichen.

8. Verfahren zur Herstellung einer Zusammensetzung mit der Eigenschaft, Tγ9δ2-Lymphozyten selektiv zu hemmen, wobei in der Zusammensetzung wenigstens eine Verbindung nach Anspruch 1 oder 2 in einer Menge enthalten ist, die die Tγ9δ2-Lymphozyten selektiv hemmen kann, wenn die genannte Zusammensetzung mit einem biologischen Milieu in Kontakt gebracht wird, das Tγ9δ2-Lymphozyten enthält.

9. Verfahren zur Herstellung einer therapeutischen Zusammensetzung für eine präventive oder kurative Behandlung einer Pathologie, bei der eine Aktivierung von Tγ9δ2-Lymphozyten induziert wird, bei dem wenigstens eine Verbindung nach Anspruch 1 oder 2 verwendet wird.

10. Verfahren zur Herstellung einer therapeutischen Zusammensetzung zur Verabreichung an einen Primaten zur präventiven oder kurativen Behandlung einer Pathologie, bei der eine Aktivierung von Tγ9δ2-Lymphozyten induziert wird, bei dem wenigstens eine Verbindung nach Anspruch 1 oder 2 verwendet wird.

11. Verfahren zur Herstellung einer therapeutischen Zusammensetzung zur Verabreichung an einen Primaten für die präventive oder kurative Behandlung einer Pathologie, bei der eine Aktivierung von Tγ9δ2-Lymphozyten induziert wird, bei dem wenigstens eine Verbindung nach Anspruch 1 oder 2 in Kontakt mit peripherem Blut oder auf topischem Wege verwendet wird.

12. Verfahren zur Herstellung einer therapeutischen Zusammensetzung zur Verabreichung an einen Primaten zur präventiven oder kurativen Behandlung von Parasitosen, die zu der Gruppe bestehend aus Malaria, viszeraler Leishmaniose und Toxoplasmose gehören, bei dem wenigstens eine Verbindung nach Anspruch 1 oder 2 verwendet wird.

13. Verfahren zur Herstellung einer therapeutischen Zusammensetzung zur Verabreichung an einen Primaten zur präventiven oder kurativen Behandlung einer Autoimmunkrankheit, die eine Aktivierung von Tγ9δ2-Lymphozyten impliziert, bei dem wenigstens eine Verbindung nach Anspruch 1 oder 2 verwendet wird.

14. Verfahren zur Herstellung einer therapeutischen Zusammensetzung zur Verabreichung an einen Primaten für die präventive oder kurative Behandlung einer Autoimmunkrankheit, die eine Aktivierung von Tγ9δ2-Lymphozyten impliziert, ausgewählt aus Plaquesklerose und Behçet-Erkrankung, bei dem wenigstens eine Verbindung nach Anspruch 1 oder 2 verwendet wird.

15. Verfahren zur Herstellung einer therapeutischen Zusammensetzung zur Verabreichung an einen Primaten für die präventive oder kurative Behandlung einer bakteriellen Pathologie, die zu der Gruppe bestehend aus Bruzellose, Tularemie, Salmonellosen, Tuberkulosen und Ehrlichiose gehört, bei dem wenigstens eine Verbindung nach Anspruch 1 oder 2 verwendet wird.

16. Verfahren zum selektiven Hemmen von Tγ9δ2-Lymphozyten in einem extrakorporalen Milieu, in dem die Tγ9δ2-Lymphozyten in dem extrakorporalen Milieu mit wenigstens einer Verbindung der folgenden Formel in Kontakt gebracht werden:
CH₃-R₁-(CH₂)₂-R₂ (I)
wobei R₁ aus den folgenden Funktionsgruppen ausgewählt ist: 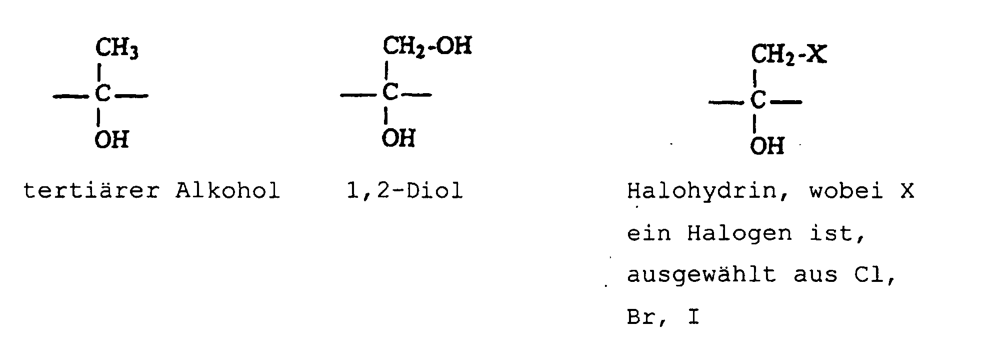 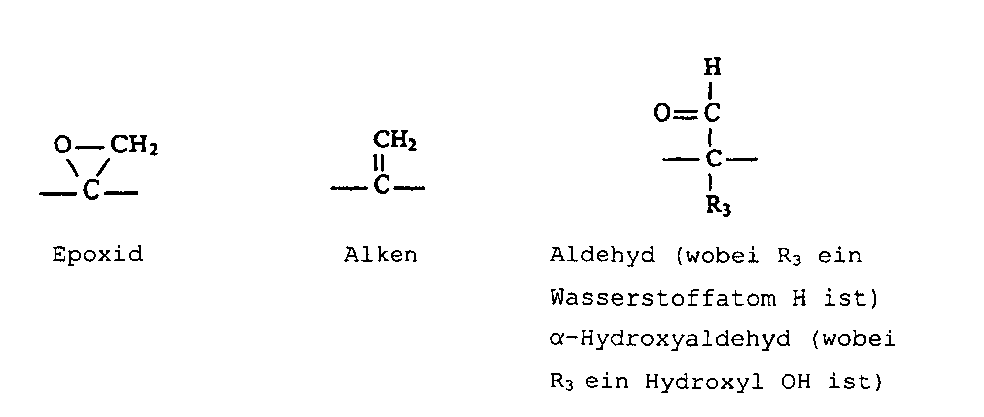 und R₂ aus einer der folgenden Gruppierungen ausgewählt ist: 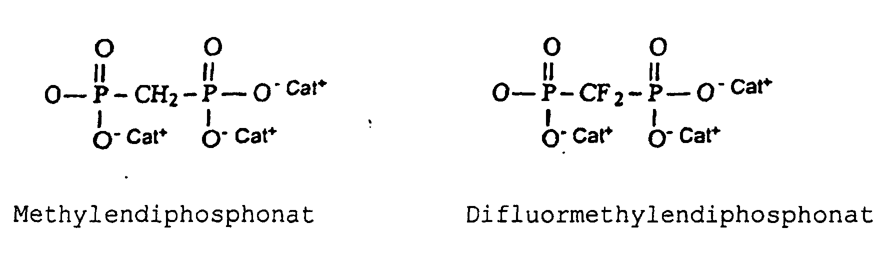 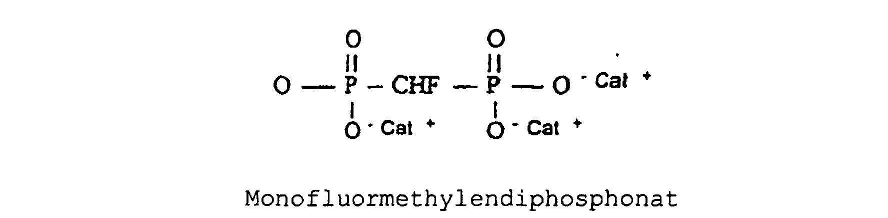 wobei Cat+ ein oder mehrere organische(s) oder mineralische(s) Kation(en) repräsentiert (einschließlich des Protons), die mit denen in derselben Zusammensetzung identisch sind oder sich von ihnen unterscheiden.

17. Verfahren nach Anspruch 16, **dadurch gekennzeichnet, dass** wenigstens eine Verbindung nach Formel (I) mit einer Konzentration in dem Milieu verwendet wird, die eine Hemmung der polyklonalen Ausbreitung von Tγ9δ2-Lymphozyten erzielt.

## Claims

1. Compounds of formula:
CH₃-R₁- (CH₂)₂-R₂ (I)
in which R₁ is chosen from the following functions: 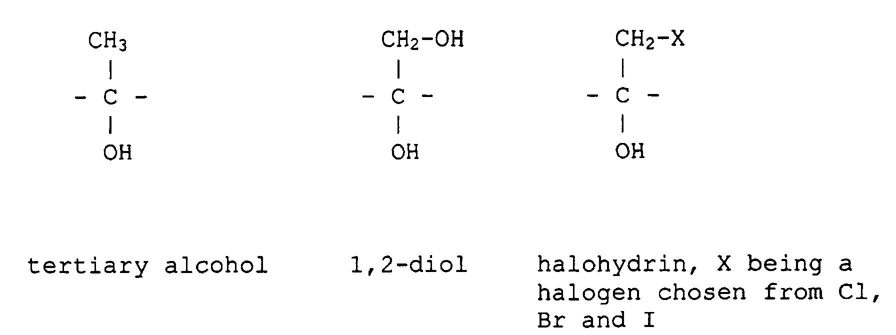 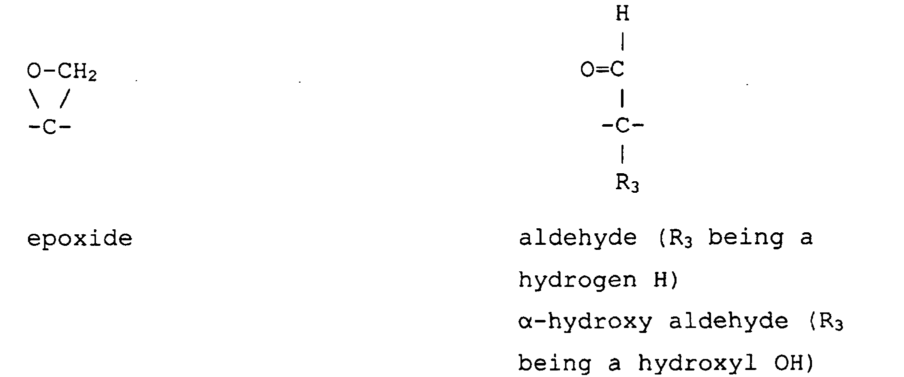 and R₂ is chosen from one of the following groups: 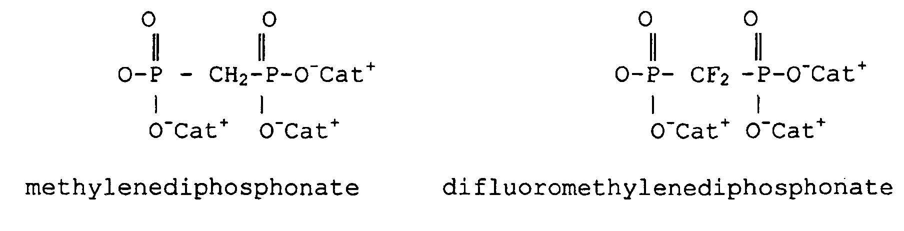 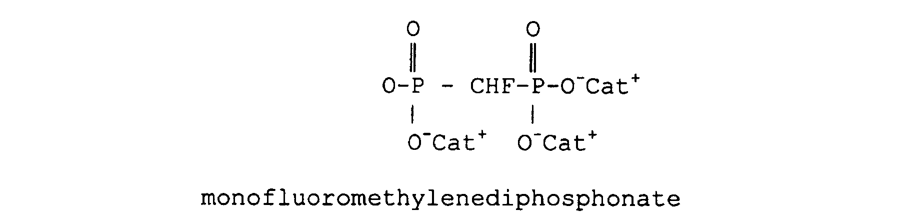 in which Cat⁺ represents one (or more) organic or mineral cation(s) (including a proton), which may be identical or different in the same compound.

2. Compounds of formula:
CH₃-R₁-(CH₂)₂-R₂ (I)
in which R₁ is chosen from the following functions: 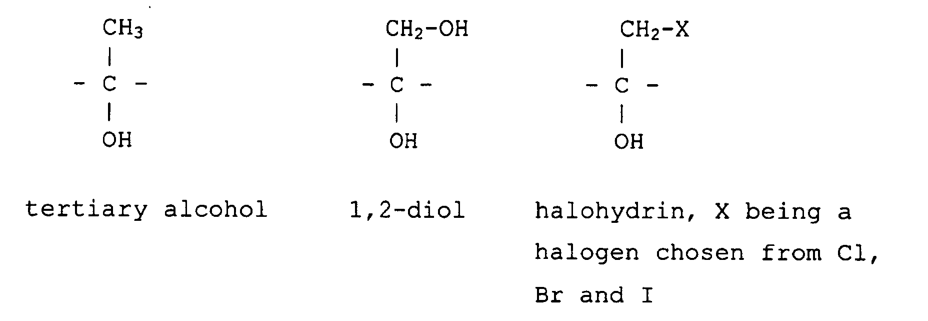 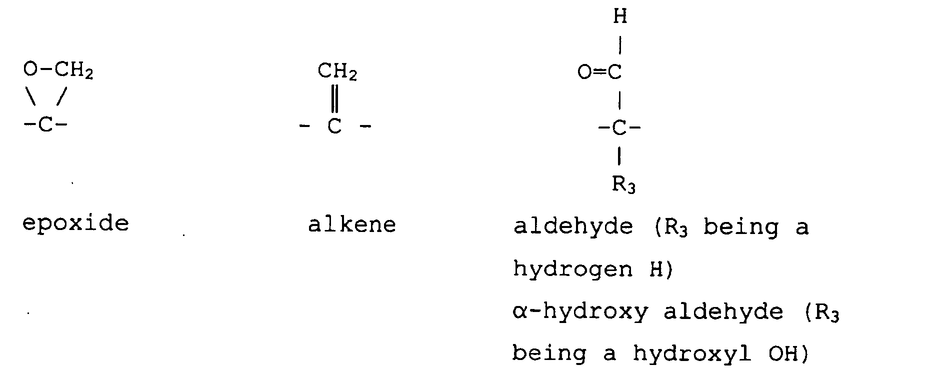 and R₂ is chosen from one of the following groups: 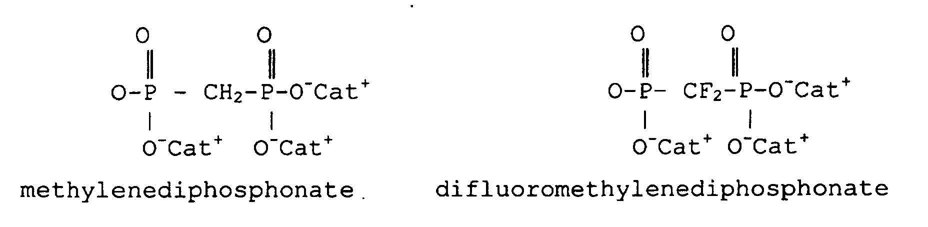 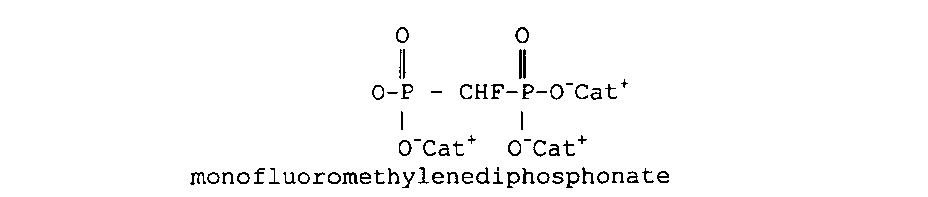 in which Cat⁺ represents one (or more) organic or mineral cation(s) (including a proton), which may be identical or different in the same compound,
for their uses as therapeutically active substances.

3. Compounds according to Claim 1, for their use as therapeutically active substances.

4. Compounds according to either of Claims 1 and 2, for their use in a therapeutic composition intended to be administered to a primate for the preventive or curative treatment of a pathology involving activation of the γ9δ2 T lymphocytes.

5. Therapeutic or diagnostic composition, **characterized in that** it comprises at least one compound according to either of Claims 1 and 2.

6. Composition according to Claim 5, **characterized in that** it comprises an amount of at least one compound according to either of Claims 1 and 2 that can be administered to a primate.

7. Composition according to Claim 5, **characterized in that** it comprises an amount of at least one compound according to either of Claims 1 and 2 that can be administered to a primate in contact with peripheral blood or via the topical route.

8. Process for manufacturing a composition having the property of selectively inhibiting γ9δ2 T lymphocytes, in which at least one compound according to either of Claims 1 and 2 is incorporated into the composition in an amount that can selectively inhibit γ9δ2 T lymphocytes when the said composition is placed in contact with a biological medium containing γ9δ2 T lymphocytes.

9. Process for manufacturing a therapeutic composition for the preventive or curative treatment of a pathology inducing activation of the γ9δ2 T lymphocytes, in which at least one compound according to either of Claims 1 and 2 is used.

10. Process for manufacturing a therapeutic composition intended to be administered to a primate for the preventive or curative treatment of a pathology inducing activation of the γ9δ2 T lymphocytes, in which at least one compound according to either of Claims 1 and 2 is used.

11. Process for manufacturing a therapeutic composition intended to be administered to a primate for the preventive or curative treatment of a pathology inducing activation of the γ9δ2 T lymphocytes, in which at least one compound according to either of Claims 1 and 2 is used in contact with peripheral blood or via the topical route.

12. Process for manufacturing a therapeutic composition intended to be administered to a primate for the preventive or curative treatment of parasitoses belonging to the group formed by malaria, visceral leishmaniasis and toxoplasmosis, in which at least one compound according to either of Claims 1 and 2 is used.

13. Process for manufacturing a therapeutic composition intended to be administered to a primate for the preventive or curative treatment of an autoimmune disease involving activation of the γ9δ2 T lymphocytes, in which at least one compound according to either of Claims 1 and 2 is used.

14. Process for manufacturing a therapeutic composition intended to be administered to a primate for the preventive or curative treatment of an autoimmune disease involving activation of the γ9δ2 T lymphocytes, chosen from multiple sclerosis and Behcet's disease, in which at least one compound according to either of Claims 1 and 2 is used.

15. Process for manufacturing a therapeutic composition intended to be administered to a primate for the preventive or curative treatment of a bacterial pathology belonging to the group formed by brucellosis, tularaemia, salmonellosis, tuberculosis and ehrlichiosis, in which at least one compound according to either of Claims 1 and 2 is used.

16. Process for selectively inhibiting γ9δ2 T lymphocytes in an extracorporeal medium, in which the γ9δ2 T lymphocytes are placed in contact, in the said extracorporeal medium, with at least one compound of formula:
CH₃-R₁-(CH₂ )₂-R₂ (I)
in which R₁ is chosen from the following functions: 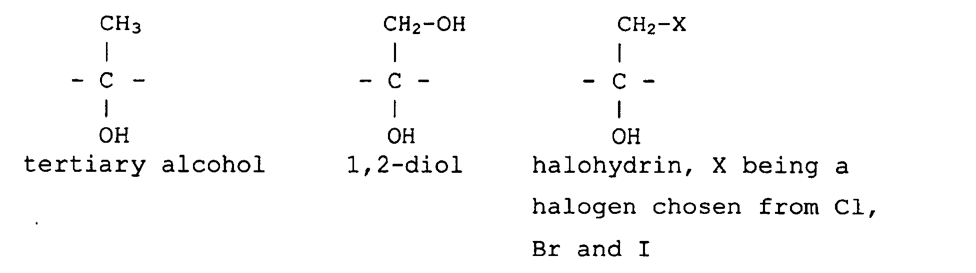 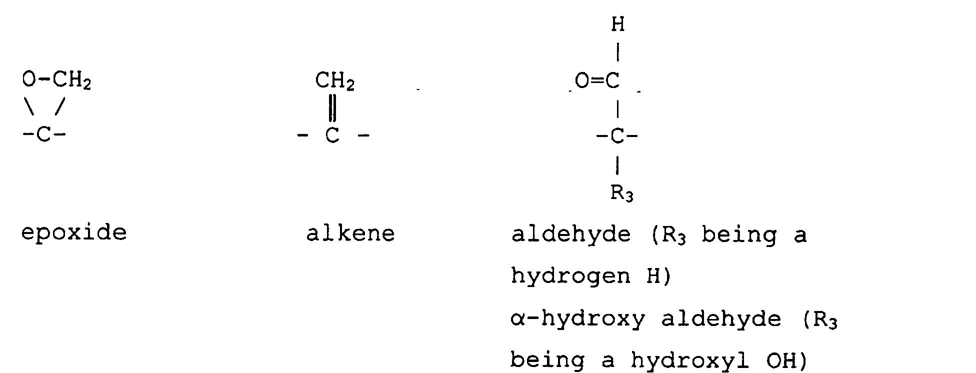 and R₂ is chosen from one of the following groups: 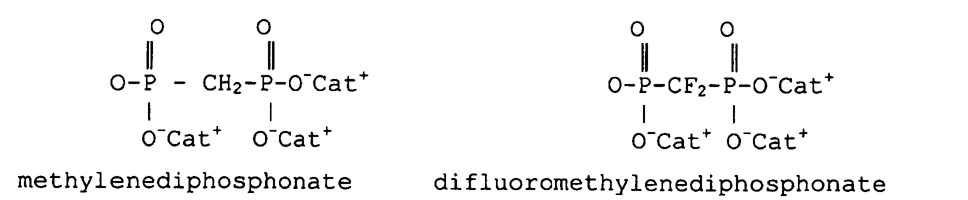 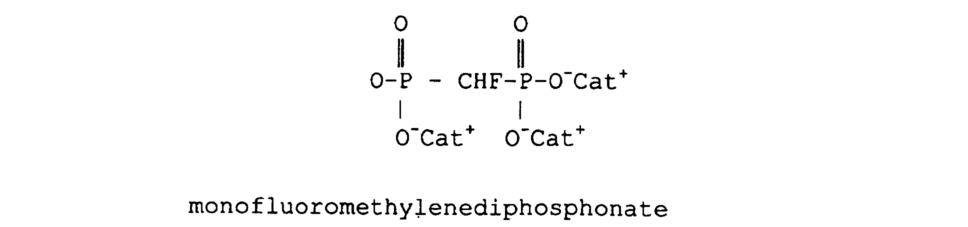 in which Cat⁺ represents one (or more) organic or mineral cation(s) (including a proton), which may be identical or different in the same compound.

17. Process according to Claim 16, **characterized in that** at least one compound according to formula (I) is used at a concentration in the medium that affords inhibition of the polyclonal proliferation of γ9δ2 T lymphocytes.
